# EUROPEAN PATENT APPLICATION

(11) **EP 3 597 191 A1**
(43) Date of publication of application: **22.01.2020**
(21) Application number: 19164801.3
(22) Date of filing: 06.12.2013
(51) Int. Cl.: A61K 31/4439, A61P 35/00, A61K 31/37, A61K 31/454, A61K 31/573, A61K 31/616, A61K 31/727, A61K 39/00

(54) **COMPOSITIONS COMPRISING ANTI-CD38 ANTIBODIES AND LENALIDOMIDE FOR USE IN TREATING CANCER**

(30) Priority: 07.12.2012 US 201261734524 P; 26.02.2013 US 201361769247 P; 04.04.2013 US 201361808372 P
(62) Divisional of application: 13861470.6
(71) Applicant: Sanofi, 75008 Paris (FR); The Regents of the University of California, Oakland, CA 94607 (US)
(72) Inventor: TOMKINSON, Blake, BRIDGEWATER, NJ New Jersey 08807 (US); HANN, Byron, C., San Francisco, CA California 94143 (US); MARTIN, III, Thomas, G., San Francisco, CA California 94143 (US); AFTAB, Blake, T., San Francisco, CA California 94158 (US)
(74) Representative: Lavoix

(57) **Abstract**

Disclosed herein are compositions and kits which comprise anti-CD38 antibodies and lenalidomide compounds. Also disclosed are methods for treating cancers, such as multiple myeloma, in subjects with the compositions and kits.

## Description

### CROSS-REFERENCE TO RELATED APPLICATIONS

This application claims the benefit of U.S. Application No. 61/734,524, filed 7 December 2012, U.S. Application No. 61/769,247, filed 26 February 2013, and U.S. Application No. 61/808,372, filed 4 April 2013, all of which are herein incorporated by reference in their entirety.

### REFERENCE TO A SEQUENCE LISTING SUBMITTED VIA EFS-WEB

The content of the ASCII text file of the sequence listing named "20130404_034543_001P3_seq" which is 56.7 kb in size was created on 4 April 2013 (technically modified Thursday, April 04, 2013, 11:12:48 AM, and created Friday, July 26, 2013, 12:23:47 PM) and electronically submitted via EFS-Web herewith the application is incorporated herein by reference in its entirety.

### BACKGROUND OF THE INVENTION

### 1. FIELD OF THE INVENTION

The field of the present invention relates to anti-CD38 antibodies, lenalidomide, and cancer treatments.

### 2. DESCRIPTION OF THE RELATED ART

Multiple myeloma (MM) is a B cell malignancy. In MM, abnormal plasma cells accumulate in the bone marrow where they interfere with the production of normal cells. Current therapy of MM includes administration of proteasome inhibitors such as bortezomib, immunomodulatory drugs such as lenalidomide and thalidomide, and chemotherapy such as melphalan and prednisone. While these agents have improved survival in multiple myeloma, invariably resistance becomes problematic and patients succumb from their illness. Multiple myeloma thus remains ultimately fatal, with a median survival of approximately 3 to 5 years only.

CD38 is expressed on malignant plasma cells. CD38 is a 45 kD type II transmembrane glycolprotein with a long C-terminal extracellular domain and a short N-terminal cytoplasmic domain. The CD38 protein is a bifunctional ectoenzyme that can catalyze the conversion of NAD⁺ into cyclic ADP-ribose (cADPR) and also hydrolyze cADPR into ADP-ribose. CD38 is up-regulated and has been implicated in many hematopoietic malignancies.

Thus, some proposed MM treatments include the administration of anti-CD38 antibodies. See, for example, WO 2012/041800; de Weers et al. (2011) J Immunol 186:1840-1848; and Van der Veer et al. (2011) Haematologica 96(2):284-290. Unfortunately, like various drugs and chemotherapies, not all antibodies are the same and not all antibodies against the same antigen exhibit the same activities.

There is thus a need for new and efficacious treatments for extending survival and improving outcome of treatments of multiple myeloma, and more generally of blood cancers.

### DESCRIPTION OF THE DRAWINGS

Both the foregoing general description and the following detailed description are exemplary and explanatory only and are intended to provide further explanation of the invention as claimed. The accompanying drawings are included to provide a further understanding of the invention and are incorporated in and constitute part of this specification, illustrate several embodiments of the invention, and together with the description serve to explain the principles of the invention.

This invention is further understood by reference to the drawings wherein:
Figure 1A shows the growth rate of tumors in xenograft models implanted with H929 cells (H929 models).
Figure 1B shows the growth rate of tumors in xenograft models implanted with RPMI8226 cells (RPMI8226 models).
Figure 2A shows the tumor volume of tumors in RPMI8226 models after treatment with the indicated dose of hu38SB19 at the indicated times (arrows).
Figure 2B shows the body weight of the RPMI8226 models after treatment with the indicated dose of hu38SB19 at the indicated times (arrows).
Figure 3A shows the tumor volume of tumors in RPMI8226 models after treatment with the indicated dose of lenalidomide at the indicated times (arrows).
Figure 3B shows the body weight of the RPMI8226 models after treatment with the indicated dose of lenalidomide at the indicated times (arrows).
Figure 4A shows the tumor volume of tumors in RPMI8226 models after treatment with the indicated dose of hu38SB19 at the indicated times (top arrows) and the indicated dose of lenalidomide at the indicated times (bottom arrows).
Figure 4B shows the body weight of the RPMI8226 models after treatment with the indicated dose of hu38SB19 at the indicated times (top arrows) and the indicated dose of lenalidomide at the indicated times (bottom arrows).
Figure 5A shows the tumor volume of tumors in H929 models after treatment with the indicated dose of hu38SB19 at the indicated times (arrows).
Figure 5B shows the body weight of the H929 models after treatment with the indicated dose of hu38SB19 at the indicated times (arrows).
Figure 6A shows the tumor volume of tumors in H929 models after treatment with the indicated dose of hu38SB19 at the indicated times (arrows).
Figure 6B shows the body weight of the H929 models after treatment with the indicated dose of hu38SB19 at the indicated times (arrows).
Figure 7A shows the tumor volume of tumors in H929 models after treatment with the indicated dose of hu38SB19 at the indicated times (arrows).
Figure 7B shows the body weight of the H929 models after treatment with the indicated dose of hu38SB19 at the indicated times (arrows).
Figure 8A shows the tumor volume of tumors in H929 models after treatment with the indicated dose of lenalidomide at the indicated times (arrows).
Figure 8B shows the body weight of the H929 models after treatment with the indicated dose of lenalidomide at the indicated times (arrows).
Figure 9A is a graph showing the mean wet tumor weights of the RPMI8226 models after the indicated treatment with lenalidomide and/or hu38SB19 (mAb).
Figure 9B is a graph showing the median wet tumor weights of the RPMI8226 models after the indicated treatment with lenalidomide and/or hu38SB19 (mAb).
Figure 10A shows the tumor volume of tumors in H929 models after treatment with the indicated dose of hu38SB19 at the indicated times (top arrows) and the indicated dose of lenalidomide at the indicated times (bottom arrows).
Figure 10B shows the body weight of the H929 models after treatment with the indicated dose of hu38SB19 at the indicated times (top arrows) and the indicated dose of lenalidomide at the indicated times (bottom arrows).
Figure 11A is a graph showing the mean wet tumor weights of the H929 models after the indicated treatment with lenalidomide and/or hu38SB19 (mAb).
Figure 11B is a graph showing the median wet tumor weights of the H929 models after the indicated treatment with lenalidomide and/or hu38SB19 (mAb).

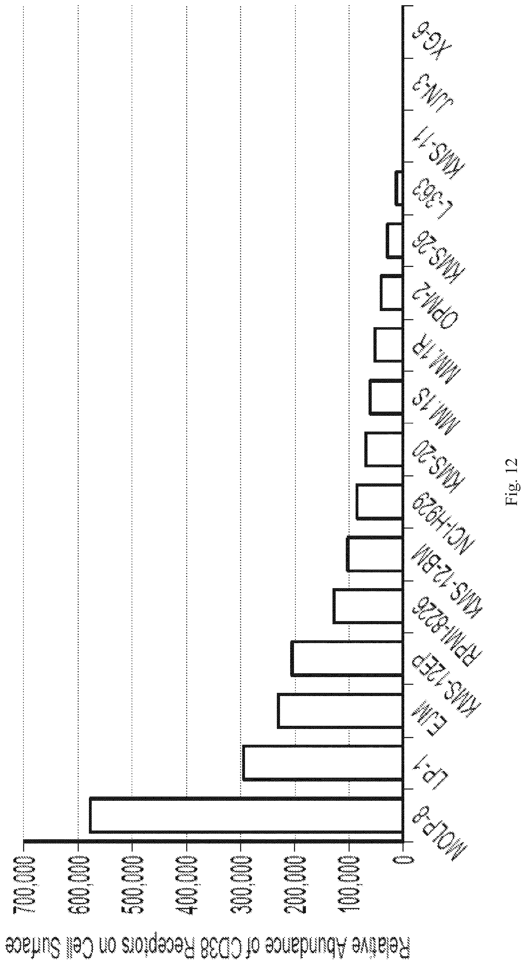

Figure 12 is a graph showing the cell surface density of CD38 in multiple myeloma cell lines.
Figure 13 is a graph showing that hu38SB19 inhibits RPMI-8226 tumor growth as a single-agent, i.e., as the sole active ingredient.
Figure 14 is a graph showing that treatment with both hu38SB19 and lenalidomide inhibits growth of RPMI-8226 tumors.

### SUMMARY OF THE INVENTION

In some embodiments, the present invention relates to a method of treating a cancer in a subject which comprises administering one or more anti-CD38 antibodies and one or more lenalidomide compounds to the subject. In some embodiments, the cancer is a hematological malignancy. In some embodiments, the cancer is multiple myeloma. In some embodiments, the cancer is a relapsed multiple myeloma or a refractory multiple myeloma. In some embodiments, the one or more lenalidomide compounds is lenalidomide. In some embodiments, the one or more anti-CD38 antibodies are administered in an effective amount, preferably a synergistic amount. In some embodiments, the one or more anti-CD38 antibodies and/or the one or more lenalidomide compounds are administered in a therapeutically effective amount. In some embodiments, at least one of the one or more anti-CD38 antibodies is capable of killing a CD38+ cell by apoptosis, antibody-dependent cell-mediated cytotoxicity (ADCC), and complement-dependent cytotoxicity (CDC). In some embodiments, the antibody is hu38SB19. In some embodiments, at least one of the one or more anti-CD38 antibodies comprises one or more complementarity-determining region having an amino acid sequence selected from the group consisting of SEQ ID NOs: 13, 14, 81, 15, 16, 17, 18, 1, 2, 3, 4, 5, 6, 7, 8, 9, 10, 11, 12, 19, 20, 21, 22, 23, 24, 25, 26, 27, 28, 29, 30, 31, 32, 33, 34, 35 and 36. In some embodiments, at least one of the one or more anti-CD38 antibodies is selected from the group consisting of: a) an antibody comprising a heavy chain comprising three sequential CDRs having amino acid sequences consisting of SEQ ID NOs: 13, 15 and either SEQ ID NO: 14 or SEQ ID NO: 81, and a light chain comprising three sequential CDRs having amino acid sequences consisting of SEQ ID NOs: 16, 17 and 18; b) an antibody comprising a heavy chain comprising three sequential CDRs having amino acid sequences consisting of SEQ ID NOs: 25, 26 and 27, and a light chain comprising three sequential CDRs having amino acid sequences consisting of SEQ ID NOs: 28, 29 and 30; c) an antibody comprising a heavy chain comprising three sequential CDRs having amino acid sequences consisting of SEQ ID NOs: 1, 2 and 3, and a light chain comprising three sequential CDRs having amino acid sequences consisting of SEQ ID NOs: 4, 5 and 6; d) an antibody comprising a heavy chain comprising three sequential CDRs having amino acid sequences consisting of SEQ ID NOs: 7, 8 and 9, and a light chain comprising three sequential CDRs having amino acid sequences consisting of SEQ ID NOs: 10, 11 and 12; e) an antibody comprising a heavy chain comprising three sequential CDRs having amino acid sequences consisting of SEQ ID NOs: 19, 20 and 21, and a light chain comprising three sequential CDRs having amino acid sequences consisting of SEQ ID NOs: 22, 23 and 24; and f) an antibody comprising a heavy chain comprising three sequential CDRs having amino acid sequences consisting of SEQ ID NOs: 31, 32 and 33, and a light chain comprising three sequential CDRs having amino acid sequences consisting of SEQ ID NOs: 34, 35 and 36. In some embodiments, the antibody comprises a heavy chain having a VH variable region represented by SEQ ID NO: 66, and a light chain having a VL variable region represented by either SEQ ID NO: 62 or SEQ ID NO: 64. In some embodiments, the antibody comprises a heavy chain having a VH variable region represented by SEQ ID NO: 72, and a light chain having a VL variable region represented by either SEQ ID NO: 68 or SEQ ID NO: 70. In some embodiments, the one or more anti-CD38 antibodies are administered intravenously. In some embodiments, the one or more lenalidomide compounds are administered orally. In some embodiments, the one or more anti-CD38 antibodies and the one or more lenalidomide compounds are administered sequentially. In some embodiments, the method further comprises administering a dexamethasone compound, preferably dexamethasone, to the subject. In some embodiments, the dexamethasone compound is administered orally. In some embodiments, the dexamethasone compound is administered at a low dose. In some embodiments, the one or more anti-CD38 antibodies, the one or more lenalidomide compounds, and the dexamethasone compound are administered sequentially. In some embodiments, the method further comprises administering an anti-coagulation agent to the subject. In some embodiments, the anti-coagulation agent is selected from the group consisting of aspirin, warfarin, and low molecular weight heparin. In some embodiments, the one or more anti-CD38 antibodies, the one or more lenalidomide compounds, and the anti-coagulation agent are administered sequentially.

In some embodiments, the present invention relates to a composition comprising a) at least one anti-CD38 antibody, preferably the antibody is capable of killing a CD38+ cell by apoptosis, antibody-dependent cell-mediated cytotoxicity (ADCC), and complement-dependent cytotoxicity (CDC); and b) at least one lenalidomide compound, preferably lenalidomide; and, optionally c) a dexamethasone compound, preferably dexamethasone; and, optionally d) an anti-coagulation agent. In some embodiments, the present invention relates to a composition comprising a) at least one anti-CD38 antibody; and b) at least one lenalidomide compound; and, optionally i) a dexamethasone compound; and/or ii) an anti-coagulation agent. In some embodiments, the antibody is capable of killing a CD38+ cell by apoptosis, antibody-dependent cell-mediated cytotoxicity (ADCC), and complement-dependent cytotoxicity (CDC). In some embodiments, the antibody is hu38SB19. In some embodiments, the lenalidomide compound is lenalidomide. In some embodiments, the dexamethasone compound is dexamethasone.

In some embodiments, the present invention is directed to a kit comprising a) a first composition comprising at least one anti-CD38 antibody, preferably the antibody is capable of killing a CD38+ cell by apoptosis, antibody-dependent cell-mediated cytotoxicity (ADCC), and complement-dependent cytotoxicity (CDC); and b) a second composition comprising at least one lenalidomide compound, preferably lenalidomide. In some embodiments, the compositions in the kit are packaged for sequential administration to a subject. In some embodiments, the antibody is hu38SB19. In some embodiments, the kit further includes a dexamethasone compound, preferably dexamethasone, and/or an anti-coagulation agent. In some embodiments, the dexamethasone compound and/or the anti-coagulation agent are packaged sequential administration to a subject.

In some embodiments, the present invention is directed to a kit comprising at least one anti-CD38 antibody capable of killing a CD38+ cell by apoptosis, antibody-dependent cell-mediated cytotoxicity (ADCC), and complement-dependent cytotoxicity (CDC), packaged together with a label having one or more messages that the at least one anti-CD38 antibody shall be administered in combination with lenalidomide, and optionally with dexamethasone and/or an anti-coagulation agent. In some embodiments, the antibody is hu38SB19. In some embodiments, the kit further includes a dexamethasone compound, preferably dexamethasone, and/or an anti-coagulation agent. In some embodiments, the dexamethasone compound and/or the anti-coagulation agent are packaged sequential administration to a subject.

In some embodiments, the present invention is directed to a combination of: (i) at least one anti-CD38 antibody, preferably the antibody is capable of killing a CD38+ cell by apoptosis, antibody-dependent cell-mediated cytotoxicity (ADCC), and complement-dependent cytotoxicity (CDC); and (ii) at least one lenalidomide compound, preferably lenalidomide; and, optionally (iii) a dexamethasone compound, preferably dexamethasone; and, optionally (iv) an anti-coagulation agent. In some embodiments, the present invention relates to a combination comprising a) at least one anti-CD38 antibody; and b) at least one lenalidomide compound; and, optionally i) a dexamethasone compound; and/or ii) an anti-coagulation agent. In some embodiments, the antibody is capable of killing a CD38+ cell by apoptosis, antibody-dependent cell-mediated cytotoxicity (ADCC), and complement-dependent cytotoxicity (CDC). In some embodiments, the antibody is hu38SB19. In some embodiments, the lenalidomide compound is lenalidomide. In some embodiments, the dexamethasone compound is dexamethasone. In some embodiments, the combination is for sequential use in the treatment of a hematological malignancy, preferably multiple myeloma.

In some embodiments, the present invention is directed to use of (i) at least one anti-CD38 antibody, preferably the antibody is capable of killing a CD38+ cell by apoptosis, antibody-dependent cell-mediated cytotoxicity (ADCC), and complement-dependent cytotoxicity (CDC); and (ii) at least one lenalidomide compound, preferably lenalidomide; and, optionally (iii) a dexamethasone compound, preferably dexamethasone; and, optionally (iv) an anti-coagulation agent for the treatment of a hematological malignancy, preferably multiple myeloma. In some embodiments, the present invention relates to use of a) at least one anti-CD38 antibody; and b) at least one lenalidomide compound; and, optionally i) a dexamethasone compound; and/or ii) an anti-coagulation agent for the treatment of a hematological malignancy, preferably multiple myeloma. In some embodiments, the antibody is capable of killing a CD38+ cell by apoptosis, antibody-dependent cell-mediated cytotoxicity (ADCC), and complement-dependent cytotoxicity (CDC). In some embodiments, the antibody is hu38SB19. In some embodiments, the lenalidomide compound is lenalidomide. In some embodiments, the dexamethasone compound is dexamethasone.

In some of the various embodiments of the present invention, the subject to be treated is mammalian. In some of the various embodiments of the present invention, the subject to be treated is a test animal such as a mouse. In some of the various embodiments of the present invention, the subject to be treated is human.

### DETAILED DESCRIPTION OF THE INVENTION

The present invention relates to methods of treating a cancer in a subject which comprises administering one or more anti-CD38 antibodies and one or more lenalidomide jurors will compounds to the subject. As used herein, "treat" or "treating" means to alleviate symptoms, eliminate the causation of the symptoms either on a temporary or permanent basis, or to prevent or slow the appearance of symptoms of the named disorder or condition. As disclosed herein, the efficacy of a lenalidomide compound is considerably improved when administered in conjunction with one or more anti-CD38 antibodies according to the present invention. In fact, the administration of one or more anti-CD38 antibodies which exhibit (a) the capability of killing a CD38⁺ cell by apoptosis, (b) antibody-dependent cell-mediated cytotoxicity (ADCC), and (c) complement-dependent cytotoxicity (CDC) is believed to considerably improve the efficacy of lenalidomide compounds in the treatment of hematological malignancies, including MM, to a degree that is unexpectedly more than other anti-CD38 antibodies which do not exhibit all three (a)-(c) activities. Therefore, in some embodiments, the one or more anti-CD38 antibodies are capable of (a) killing a CD38⁺ cell by apoptosis, (b) antibody-dependent cell-mediated cytotoxicity (ADCC), and (c) complement-dependent cytotoxicity (CDC). In some embodiments, the one or more anti-CD38 antibodies and/or the one or more lenalidomide compounds are administered in a therapeutically effective amount. As used herein, a "therapeutically effective amount" of a substance refers to an amount of that substance that results in the alleviation of one or more symptoms, elimination of the causation of the symptoms either on a temporary or permanent basis, and/or the prevention or reduction in the appearance of symptoms of the named disorder or condition in the majority of subjects afflicted with and similarly treated for the named disease or disorder.

In some embodiments, the cancer is one in which CD38 is expressed by the malignant cells. In some embodiments, the cancer is a hematological malignancy of the blood, bone marrow, and/or lymph nodes. In some embodiments, the cancer is a blood cancer. Blood cancers include myeloma, lymphoma and leukemia. The blood cancer might, for instance, be selected from the group consisting of multiple myeloma, non-Hodgkin's lymphoma, Hodgkin's lymphoma, hairy cell leukemia, chronic lymphocytic leukemia, chronic myeloid leukemia, acute myeloid leukemia, and acute lymphocytic leukemia. In some embodiments, the cancer is multiple myeloma (MM). In some embodiments, the cancer is a relapse MM or refractory MM. As used herein, relapsed MM refers to clinically active MM after a period of remission and refractory MM refers to progressive or stable disease while being treated or progressive disease within 3 months of the last does of the prior treatment. See Dimopoulos et al. (2010) Eur J Haematology 88:1-15.

In some embodiments, the subject is mammalian, preferably human. In some embodiments, the subject is an adult human, e.g., at least 18 years. In some embodiments, the subject is in need of treatment for the cancer. In some embodiments, the subject has been diagnosed as having the cancer. In some embodiments, the cancer is in partial or complete remission, however, the one or more lenalidomide compounds and the one or more anti-CD38 antibodies are administered to the subject so as to reduce the likelihood of relapse. In some embodiments, the subject has a Karnofsky performance status equal or superior to 60%. The Karnofsky status runs from 100 to 0, where 100 is "perfect" health and 0 is death (Karnofsky and Burchenal, 1949, "The Clinical Evaluation of Chemotherapeutic Agents in Cancer." In: MacLeod CM (Ed), Evaluation of Chemotherapeutic Agents. Columbia Univ Press). In some embodiments, the subject has undergone at least one or two prior therapies for multiple myeloma, induction therapy being considered one prior therapy. In some embodiments, the subject exhibits evidence that either the cancer progressed while the subject underwent a prior therapy, or that the subject was refractory to the prior therapy.

In some embodiments, the anti-CD38 antibodies specifically bind CD38. In some embodiments, the anti-CD38 antibodies are raised against CD38 or an epitope thereof. In some embodiments, the anti-CD38 antibodies are monoclonal antibodies. In some embodiments, one or more of the anti-CD38 antibodies according to the present invention are monoclonal antibodies as described in WO 2008/047242, which is herein incorporated by reference in its entirety. In some embodiments, one or more of the anti-CD38 antibodies are monoclonal antibodies 38SB13, 38SB18, 38SB19, 38SB30, 38SB31, and 38SB39 as described in WO 2008/047242, which is herein incorporated by reference in its entirety. In some embodiments, the one or more anti-CD38 antibodies are capable of killing CD38⁺ cells by three different cytotoxic mechanisms, induction of apoptosis, antibody-dependent cell-mediated cytotoxicity (ADCC), and complement-dependent cytotoxicity (CDC).

The term "antibody" is used herein in the broadest sense and includes monoclonal antibodies (including full length monoclonal antibodies) of any isotype such as IgG, IgM, IgA, IgD and IgE, polyclonal antibodies, multispecific antibodies, chimeric antibodies, and antibody fragments. As used herein, the prefix "anti-" when in conjunction with an antigen, indicates that the given antibody is reactive with the given antigen. An antibody reactive with a specific antigen can be generated by synthetic and/or recombinant methods such as selection of libraries of recombinant antibodies in phage or similar vectors, or by immunizing an animal with the antigen or an antigen-encoding nucleic acid.

A typical IgG antibody is comprised of two identical heavy chains and two identical light chains that are joined by disulfide bonds. Each heavy and light chain contains a constant region and a variable region. Each variable region contains three segments called "complementarity-determining regions" ("CDRs") or "hypervariable regions", which are primarily responsible for binding an epitope of an antigen. They are usually referred to as CDR1, CDR2, and CDR3, numbered sequentially from the N-terminus. The more highly conserved portions of the variable regions outside of the CDRs are called the "framework regions". As used herein, "V_{H}" or "VH" refers to the variable region of an immunoglobulin heavy chain of an antibody, including the heavy chain of an Fv, scFv, dsFv, Fab, Fab' or F(ab')₂ fragment. Reference to "V_{L}" or "VL" refers to the variable region of the immunoglobulin light chain of an antibody, including the light chain of an Fv, scFv, dsFv, Fab, Fab' or F(ab')₂ fragment.

The antibodies according to the present invention may be, e.g., murine, chimeric, and/or humanized antibodies. As used herein, a "chimeric antibody" is an antibody in which the constant region, or a portion thereof, is altered, replaced, or exchanged, so that the variable region is linked to a constant region of a different species, or belonging to another antibody class or subclass. "Chimeric antibody" also refers to an antibody in which the variable region, or a portion thereof, is altered, replaced, or exchanged, so that the constant region is linked to a variable region of a different species, or belonging to another antibody class or subclass. Methods for producing chimeric antibodies are known in the art. See e.g., Morrison, 1985, Science, 229: 1202; Oi et al., 1986, BioTechniques, 4: 214; Gillies et al., 1989, J. Immunol. Methods, 125: 191-202; U.S. Pat. Nos. 5,807,715; 4,816,567; and 4,816,397, which are incorporated herein by reference in their entireties. The term "humanized antibody", as used herein, refers to a chimeric antibody which contain minimal sequence derived from non-human immunoglobulin. The goal of humanization is a reduction in the immunogenicity of a xenogenic antibody, such as a murine antibody, for introduction into a human, while maintaining the full antigen binding affinity and specificity of the antibody. Humanized antibodies, or antibodies adapted for non-rejection by other mammals, may be produced using several technologies such as resurfacing and CDR grafting. As used herein, the resurfacing technology uses a combination of molecular modelling, statistical analysis and mutagenesis to alter the non-CDR surfaces of antibody variable regions to resemble the surfaces of known antibodies of the target host. The CDR grafting technology involves substituting the complementarity determining regions of, for example, a mouse antibody, into a human framework domain, e.g., see WO 92/22653. Humanized chimeric antibodies preferably have constant regions and variable regions other than the complementarity determining regions (CDRs) derived substantially or exclusively from the corresponding human antibody regions and CDRs derived substantially or exclusively from a mammal other than a human.

Strategies and methods for the resurfacing of antibodies, and other methods for reducing immunogenicity of antibodies within a different host, are disclosed in US Pat. No. 5,639,641, which is hereby incorporated in its entirety by reference. Antibodies can be humanized using a variety of other techniques including CDR-grafting (EP 0 239 400; WO 91/09967; US Pat. Nos. 5,530,101; and 5,585,089), veneering or resurfacing (EP 0 592 106; EP 0 519 596; Padlan E. A., 1991, Molecular Immunology 28(4/5): 489-498; Studnicka G. M. et al., 1994, Protein Engineering, 7(6): 805-814; Roguska M.A. et al., 1994, PNAS, 91: 969-973), chain shuffling (US Pat. No. 5,565,332), and identification of flexible residues (PCT/US2008/074381). Human antibodies can be made by a variety of methods known in the art including phage display methods. See also US Pat. Nos. 4,444,887, 4,716,111, 5,545,806, and 5,814,318; and international patent application publication numbers WO 98/46645, WO 98/50433, WO 98/24893, WO 98/16654, WO 96/34096, WO 96/33735, and WO 91/10741 (said references incorporated by reference in their entireties).

In some embodiments, one or more of the anti-CD38 antibodies according to the invention are capable of killing a CD38⁺ cell by apoptosis, antibody-dependent cell-mediated cytotoxicity (ADCC), and complement-dependent cytotoxicity (CDC). In some embodiments, one or more of the anti-CD38 antibodies according to the invention are capable of killing said CD38⁺ cells by apoptosis even in the absence of stroma cells or stroma-derived cytokines. These activities can be assessed as described in WO 2008/047242, which is hereby incorporated by reference in its entirety.

In some embodiments according to the invention, one or more anti-CD38 antibodies are selected from the group consisting of 38SB13, 38SB18, 38SB19, 38SB30, 38SB31, 38SB39, and antibodies cross-competing with 38SB13, 38SB18, 38SB19, 38SB30, 38SB31 or 38SB39. The hybridoma cell lines producing the 38SB13, 38SB18, 38SB19, 38SB30, 38SB31, and 38SB39 murine anti-CD38 antibodies have been deposited at the American Type Culture Collection (10801 University Bld, Manassas, VA, 20110-2209, USA), on 21 June 21 2006, under the deposit numbers PTA-7667, PTA-7669, PTA-7670, PTA-7666, PTA-7668, and PTA-7671, respectively (as described in WO 2008/047242, which is herein incorporated by reference in its entirety).

As disclosed herein, references to SEQ ID NOs refers to the sequences set forth in the Sequence Listing submitted herewith and also as recited in WO 2008/047242, which is herein incorporated by reference in its entirety. In some embodiments, the anti-CD38 antibodies according to the present invention may, for instance, comprise a heavy chain comprising three sequential CDRs having amino acid sequences represented by SEQ ID NOs: 1, 2, and 3, and a light chain comprising three sequential CDRs having amino acid sequences represented by SEQ ID NOs: 4, 5, and 6. An example of such an antibody is the 38SB13 antibody, which comprises a heavy chain having a V_{H} variable region represented by SEQ ID NO: 50, and a light chain having a V_{L} variable region represented by SEQ ID NO: 38.

In some embodiments, the anti-CD38 antibodies according to the present invention may, for instance, comprise a heavy chain comprising three sequential CDRs having amino acid sequences represented by SEQ ID NOs: 7, 8, and 9, and a light chain comprising three sequential CDRs having amino acid sequences represented by SEQ ID NOs: 10, 11, and 12. An example of such an antibody is the 38SB18 antibody, which comprises a heavy chain having a V_{H} variable region represented by SEQ ID NO: 52 and a light chain having a V_{L} variable region represented by SEQ ID NO: 40.

In some embodiments, the anti-CD38 antibodies according to the present invention may, for instance, comprise a heavy chain comprising three sequential CDRs having amino acid sequences represented by SEQ ID NO: 13, SEQ ID NO: 15 and either SEQ ID NO: 14 or SEQ ID NO: 81, and a light chain comprising three sequential CDRs having amino acid sequences represented by SEQ ID NOs: 16, 17, and 18. An example of such an antibody is the 38SB19 antibody, which comprises a heavy chain having a V_{H} variable region represented by SEQ ID NO: 54 and a light chain having a V_{L} variable region represented by SEQ ID NO: 42. Specific examples of humanized versions of 38SB19 (hu38SB19) include antibodies comprising a heavy chain having a V_{H} variable region represented by SEQ ID NO: 66, and a light chain having a V_{L} variable region represented by either SEQ ID NO: 62 or SEQ ID NO: 64. hu38SB19 is a humanized anti-CD38 antibody currently undergoing clinical evaluation in CD38-positive hematologic malignancies, including multiple myeloma. Previous and current studies demonstrate that the anti-myeloma activity associated with this agent involve mechanisms of ADCC, and CDC, as well as novel, direct apoptotic and anti-ADP-ribosyl cyclase activity. See Marie-Cécile Wetzel, Céline Nicolazzi, François Vallée, et al. hu38SB19: characterization of a potent phase I humanized anti-CD38 antibody for the treatment of multiple myeloma and other hematologic malignancies. AACR Annual Meeting 2013, Abstract #4735.

In some embodiments, the anti-CD38 antibodies according to the present invention may, for instance, comprise a heavy chain comprising three sequential CDRs having amino acid sequences represented by SEQ ID NOs: 19, 20, and 21, and a light chain comprising three sequential CDRs having amino acid sequences represented by SEQ ID NOs: 22, 23, and 24. An example of such an antibody is the 38SB30 antibody, which comprises a heavy chain having a V_{H} variable region represented by SEQ ID NO: 56 and a light chain having a V_{L} variable region represented by SEQ ID NO: 44.

In some embodiments, the anti-CD38 antibodies according to the present invention may, for instance, comprise a heavy chain comprising three sequential CDRs having amino acid sequences represented by SEQ ID NOs: 25, 26, and 27, and a light chain comprising three sequential CDRs having amino acid sequences represented by SEQ ID NOs: 28, 29, and 30. An example of such an antibody is the 38SB31 antibody, which comprises a heavy chain having a V_{H} variable region represented by SEQ ID NO: 58 and a light chain having a V_{L} variable region represented by SEQ ID NO: 46. Specific examples of humanized versions of 38SB31 (hu38SB31) include antibodies comprising a heavy chain having a V_{H} variable region represented by SEQ ID NO: 72, and a light chain having a V_{L} variable region represented by either SEQ ID NO: 68 or SEQ ID NO: 70.

In some embodiments, the anti-CD38 antibodies according to the present invention may, for instance, comprise a heavy chain comprising three sequential CDRs having amino acid sequences represented by SEQ ID NOs: 31, 32 and 33, and a light chain comprising three sequential CDRs having amino acid sequences represented by SEQ ID NOs: 34, 35, and 36. An example of such an antibody is the 38SB39 antibody, which comprises a heavy chain having a V_{H} variable region represented by SEQ ID NO: 60 and a light chain having a V_{L} variable region represented by SEQ ID NO: 48.

In some embodiments, the anti-CD38 antibodies according to the invention are humanized antibodies consisting of two identical heavy chains and of two identical light chains, wherein each chain consists of one constant region and of one variable region.

As used herein, a "lenalidomide compound" refers to lenalidomide ((*RS*)-3-(4-amino-1-oxo-3*H*-isoindol-2-yl)piperidine-2,6-dione) and lenalidomide derivatives. As used herein, "lenalidomide derivatives" refers to compounds which have 4-amino-1-oxo-3*H*-2-isoindolyl, i.e., 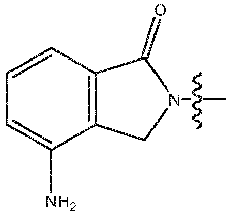 which may or may not be substituted, as part of its structural formula. For example, "lenalidomide derivatives" include those having the following formula: 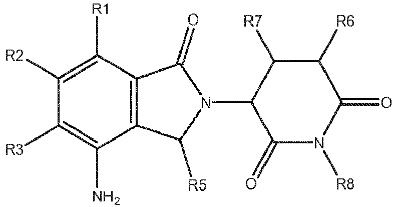 wherein R1-R8 are each independently H, a halogen, an alkyl, an alkoxy, amino, or an alkylamine, and wherein R5 may additionally be a double bonded oxygen. In some embodiments, R5 is H. In some embodiments, R8 is H. In some embodiments, both R5 and R8 are H.

In some embodiments, the one or more anti-CD38 antibodies are administered in an effective amount. As used herein, an effective amount of the one or more anti-CD38 antibodies is an amount which results in an additive or a synergistic effect with the one or more lenalidomide compounds. As used herein, a "synergistic amount" is one that results in a synergistic effect. As used herein, a "synergistic effect" refers to the effect of the combination of the one or more anti-CD38 antibodies and the one or more lenalidomide compounds which is more than their expected additive effect. In some embodiments, the one or more anti-CD38 antibodies are administered before, during, and/or after the administration of the one or more lenalidomide compounds. In some embodiments, the one or more anti-CD38 antibodies and the one or more lenalidomide compounds are co-administered in the form of a single composition, e.g., as a mixture.

Thus, in some embodiments, the present invention is directed to compositions comprising a mixture of at least one anti-CD38 antibody and at least one lenalidomide compound. In some embodiments, the mixture comprises the at least one anti-CD38 antibody in an amount that results in an additive or a synergistic effect with the at least one lenalidomide compound in a subject when both are administered. In some embodiments, the at least one anti-CD38 antibody in the mixture is one which is capable of killing a CD38⁺ cell by apoptosis, antibody-dependent cell-mediated cytotoxicity (ADCC), and complement-dependent cytotoxicity (CDC); and at least one lenalidomide compound.

For the purposes of the present invention, the methods and compositions of the present invention are not exclusively limited to those which are obtained by physical association of the anti-CD38 antibodies and the lenalidomide compound, but also to those which permit a separate administration, which can be simultaneous or spaced out over a period of time. Thus, in some embodiments, the present invention is directed to a first composition comprising the one or more anti-CD38 antibodies, and a second composition comprising one or more lenalidomide compounds. In some embodiments, the at least one anti-CD38 antibody is one which is capable of killing a CD38⁺ cell by apoptosis, antibody-dependent cell-mediated cytotoxicity (ADCC), and complement-dependent cytotoxicity (CDC); and at least one lenalidomide compound. In some embodiments, the amount of the one or more anti-CD38 antibodies provided in the first composition is one that results in an additive or a synergistic effect with the at least one lenalidomide compound in the second composition in a subject when both are administered.

In some embodiments, the first and second compositions may be packaged in a kit. Thus, in some embodiments, the present invention is directed to kits which comprise a first composition comprising the one or more anti-CD38 antibodies, and a second composition comprising one or more lenalidomide compounds. In some embodiments, the first and second composition may be mixed together before administering to a subject. In some embodiments, the first and second compositions, may be administered either simultaneously or sequentially (i.e., spaced out over a period of time) so as to obtain the maximum efficacy, additivity, synergy, or a combination thereof of the combination. In some embodiments, the present invention is directed to kits comprising at least one anti-CD38 antibody packaged together with a label having one or more messages that the anti-CD38 antibody shall or might be administered in combination with lenalidomide and optionally with dexamethasone and/or an anti-coagulation agent. The kits according to the present invention may further comprise one or more messages that the antibody shall or might be administered to a subject suffering from a blood cancer such as multiple myeloma (e.g., relapsed or refractory multiple myeloma). In some embodiments, the one or more anti-CD38 antibodies in the kits of the present invention are those which are capable of killing a CD38⁺ cell by apoptosis, antibody-dependent cell-mediated cytotoxicity (ADCC), and complement-dependent cytotoxicity (CDC).

In some embodiments, the compositions of the present invention are pharmaceutical compositions. As used herein, the term "pharmaceutical composition" refers to a composition comprising at least one active principle (e.g., an anti-CD38 antibody or a lenalidomide compound) and at least one pharmaceutically acceptable carrier. Pharmaceutically acceptable carriers are well known to the skilled in the art, and usually depend on the chosen route of administration. Pharmaceutical compositions according to the present invention may be provided in any form or formulation that is suitable for the chosen route of administration, such as e.g., a solution in case of an intravenous route of administration, e.g., capsules, pills or tablets in case of an oral route of administration, etc.

The dosage regimen of the active principles and of the pharmaceutical composition described herein can be chosen by prescribing physicians, based on their knowledge of the art, including information published by regulatory authorities. For example, lenalidomide is typically administered orally. According to the European Medicines Agency (EMA), the recommended dose of lenalidomide is 25 mg orally once daily on days 1-21 of repeated 28-day cycles. Since, however, co-administration of the one or more anti-CD38 antibodies and the one or more lenalidomide compounds results in an additive or a synergistic effect, the dosing of the lenalidomide compound may be adjusted accordingly, e.g., the dose changed and/or the dosing schedule modified. Of course, prescribing physicians might reconsider which dose and schedule to use depending on the condition and disease status of the patient and based upon clinical and laboratory findings.

As lenalidomide is approved for the treatment of MM in combination with dexamethasone, the methods and compositions of the present invention may further include dexamethasone, which is member of the glucocorticoid class of steroid drugs, and acts as an anti-inflammatory and immunosuppressant. Thus, in some embodiments, the treatment methods of the present invention further comprise administering a dexamethasone compound to the subject being treated with the one or more anti-CD38 antibodies and the one or more lenalidomide compounds. Similarly, the compositions and kits of the present invention which comprise the one or more anti-CD38 antibodies and/or the one or more lenalidomide compounds may further comprise a dexamethasone compound. As used herein, a "dexamethasone compound" refers to dexamethasone ((8*S*,9*R*,10*S*,11*S*,13*S*,14*S*,16*R*,17*R*)-9-Fluoro-11,17-dihydroxy-17-(2-hydroxyacetyl)-10,13,16-trimethyl-6,7,8,9,10,11,12,13,14,15,16,17- dodecahydro-3*H*-cyclopenta[*a*]phenanthren-3-one) and dexamethasone derivatives. As used herein, a "dexamethasone derivative" refers to a compound having the following structural formula: 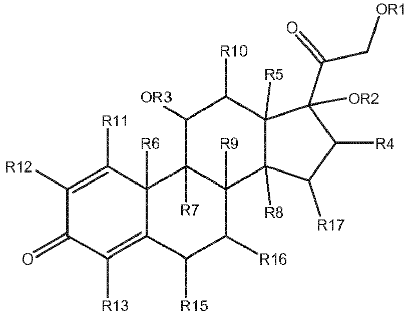 wherein R1-R17 are each independently H, a halogen, an alkyl, an alkoxy, amino, or an alkylamine. In some preferred embodiments, R1-R3 are H. In some preferred embodiments, R4-R6 are methyl. In some preferred embodiments, R7 is a halogen, preferably fluorine. In some preferred embodiments, R8 is H. In some preferred embodiments, R1-R3 are H, R4-R6 are methyl, R7 is a halogen, preferably fluorine, and R8 is H.

In some embodiments, the dexamethasone compound may be administered orally. According to the EMA, when combined with lenalidomide, the recommended dose of dexamethasone is 40 mg orally once daily on days 1-4, 9-12, and 17-20 of each 28-day cycle for the first 4 cycles of therapy and then 40 mg once daily on days 1-4 every 28 days. Prescribing physicians might also re-evaluate which dose of dexamethasone to use upon clinical and laboratory findings.

However, in some embodiments, the dexamethasone compound may be administered at a lower dose than the dose recommended for dexamethasone by the EMA. Indeed, recent studies suggest that lenalidomide plus low dose dexamethasone is associated with better short-term overall survival and with lower toxicity than lenalidomide plus high-dose dexamethasone in patients with newly diagnosed myeloma (Rajkumar et al. (2010) Lancet Onco. 11:29-37). Therefore, in some embodiments of the present invention, the dexamethasone compound is administered at low dose. The term "low dose" in this context refers to any dose that is at least 20, 30 or 40% lower that the dose of dexamethasone recommended by EMA at the date of first marketing approval of the lenalidomide plus dexamethasone combination. For instance, administration of 40 mg of dexamethasone on days 1, 8, 15, and 22 of a 28-day cycle is considered as a low dose of dexamethasone.

In some embodiments, the methods and compositions of the present invention may further include an anti-coagulation agent, such as e.g., aspirin, warfarin, low molecular weight heparin or equivalent anti-platelet therapeutic. For example, in some embodiments, the treatment methods of the present invention further comprise administering an anti-coagulation agent to the subject being treated with the one or more anti-CD38 antibodies and the one or more lenalidomide compounds. Similarly, the compositions and kits of the present invention which comprise the one or more anti-CD38 antibodies and/or the one or more lenalidomide compounds may further comprise an anti-coagulation agent.

The compositions of the present invention may be used as a medicament and/or for use in the manufacture of a medicament. In some embodiments, the compositions of the present invention may be used as a medicament and/or for use in the manufacture of a medicament for use in the treatment of a cancer such as a hematological malignancy of the blood, bone marrow, and/or lymph nodes, preferably a blood cancer.

Several documents are cited throughout the text of this specification. Each of the documents herein (including any journal article or abstract, published or unpublished patent application, issued patent, manufacturer's specifications, instructions, *etc.*) are hereby incorporated by reference. However, there is no admission that any document cited herein is indeed prior art in respect of the present invention.

In some embodiments the invention relates to
1. A method of treating a cancer in a subject which comprises administering one or more anti-CD38 antibodies and one or more lenalidomide compounds to the subject.
2. The method of item 1, wherein the cancer is a hematological malignancy.
3. The method of item 1, wherein the cancer is multiple myeloma.
4. The method of item 1, wherein the cancer is a relapsed multiple myeloma or a refractory multiple myeloma.
5. The method of item 1, wherein the one or more lenalidomide compounds is lenalidomide.
6. The method of item 1, wherein the one or more anti-CD38 antibodies are administered in an effective amount, preferably a synergistic amount.
7. The method of item 1, wherein the one or more anti-CD38 antibodies and/or the one or more lenalidomide compounds are administered in a therapeutically effective amount.
8. The method of any of items 1 to 7, wherein at least one of the one or more anti-CD38 antibodies is capable of killing a CD38⁺ cell by apoptosis, antibody-dependent cell-mediated cytotoxicity (ADCC), and complement-dependent cytotoxicity (CDC).
9. The method of any of items 1 to 7, wherein at least one of the one or more anti-CD38 antibodies comprises one or more complementarity-determining region having an amino acid sequence selected from the group consisting of SEQ ID NOs: 13, 14, 81, 15, 16, 17, 18, 1, 2, 3, 4, 5, 6, 7, 8, 9, 10, 11, 12, 19, 20, 21, 22, 23, 24, 25, 26, 27, 28, 29, 30, 31, 32, 33, 34, 35 and 36.
10. The method of any of items 1 to 7, wherein at least one of the one or more anti-CD38 antibodies is selected from the group consisting of:
   a) an antibody comprising a heavy chain comprising three sequential CDRs having amino acid sequences consisting of SEQ ID NOs: 13, 15 and either SEQ ID NO: 14 or SEQ ID NO: 81, and a light chain comprising three sequential CDRs having amino acid sequences consisting of SEQ ID NOs: 16, 17 and 18;
   b) an antibody comprising a heavy chain comprising three sequential CDRs having amino acid sequences consisting of SEQ ID NOs: 25, 26 and 27, and a light chain comprising three sequential CDRs having amino acid sequences consisting of SEQ ID NOs: 28, 29 and 30;
   c) an antibody comprising a heavy chain comprising three sequential CDRs having amino acid sequences consisting of SEQ ID NOs: 1, 2 and 3, and a light chain comprising three sequential CDRs having amino acid sequences consisting of SEQ ID NOs: 4, 5 and 6;
   d) an antibody comprising a heavy chain comprising three sequential CDRs having amino acid sequences consisting of SEQ ID NOs: 7, 8 and 9, and a light chain comprising three sequential CDRs having amino acid sequences consisting of SEQ ID NOs: 10, 11 and 12;
   e) an antibody comprising a heavy chain comprising three sequential CDRs having amino acid sequences consisting of SEQ ID NOs: 19, 20 and 21, and a light chain comprising three sequential CDRs having amino acid sequences consisting of SEQ ID NOs: 22, 23 and 24; and
   f) an antibody comprising a heavy chain comprising three sequential CDRs having amino acid sequences consisting of SEQ ID NOs: 31, 32 and 33, and a light chain comprising three sequential CDRs having amino acid sequences consisting of SEQ ID NOs: 34, 35 and 36.
11. The method of item 10, wherein said antibody comprises a heavy chain having a V_{H} variable region represented by SEQ ID NO: 66, and a light chain having a V_{L} variable region represented by either SEQ ID NO: 62 or SEQ ID NO: 64.
12. The method of item 10, wherein said antibody comprises a heavy chain having a V_{H} variable region represented by SEQ ID NO: 72, and a light chain having a V_{L} variable region represented by either SEQ ID NO: 68 or SEQ ID NO: 70.
13. The method of any of items 1 to 12, wherein the one or more anti-CD38 antibodies are administered intravenously.
14. The method of any of items 1 to 12, wherein the one or more lenalidomide compounds are administered orally.
15. The method of any of items 1 to 14, wherein the one or more anti-CD38 antibodies and the one or more lenalidomide compounds are administered sequentially.
16. The method of any of items 1 to 15, and further comprising administering a dexamethasone compound, preferably dexamethasone, to the subject.
17. The method of item 16, wherein the dexamethasone compound is administered orally.
18. The method of item 16 or 17, wherein the dexamethasone compound is administered at a low dose.
19. The method of any of items 16 to 18, wherein the one or more anti-CD38 antibodies, the one or more lenalidomide compounds, and the dexamethasone compound are administered sequentially.
20. The method of any of items 1 to 19, and further comprising administering an anti-coagulation agent to the subject.
21. The method of item 20, wherein the anti-coagulation agent is selected from the group consisting of aspirin, warfarin, and low molecular weight heparin.
22. The method of item 20 or 21, wherein the one or more anti-CD38 antibodies, the one or more lenalidomide compounds, and the anti-coagulation agent are administered sequentially.
23. A composition comprising
   a) at least one anti-CD38 antibody, preferably the antibody is capable of killing a CD38⁺ cell by apoptosis, antibody-dependent cell-mediated cytotoxicity (ADCC), and complement-dependent cytotoxicity (CDC); and
   b) at least one lenalidomide compound, preferably lenalidomide; and, optionally
   c) a dexamethasone compound, preferably dexamethasone; and, optionally
   d) an anti-coagulation agent.
24. A kit comprising
   a) a first composition comprising at least one anti-CD38 antibody, preferably the antibody is capable of killing a CD38⁺ cell by apoptosis, antibody-dependent cell-mediated cytotoxicity (ADCC), and complement-dependent cytotoxicity (CDC); and
   b) a second composition comprising at least one lenalidomide compound, preferably lenalidomide.
25. The kit of item 24, wherein the compositions are packaged for sequential administration to a subject.
26. The kit of item 24 or item 25, and further including a dexamethasone compound, preferably dexamethasone, and/or an anti-coagulation agent.
27. The kit of item 26, wherein the dexamethasone compound and/or the anti-coagulation agent are packaged sequential administration to a subject.
28. A kit comprising at least one anti-CD38 antibody capable of killing a CD38⁺ cell by apoptosis, antibody-dependent cell-mediated cytotoxicity (ADCC), and complement-dependent cytotoxicity (CDC), packaged together with a label having one or more messages that the at least one anti-CD38 antibody shall be administered in combination with lenalidomide, and optionally with dexamethasone and/or an anti-coagulation agent.
29. A combination of:
   (i) at least one anti-CD38 antibody, preferably the antibody is capable of killing a CD38+ cell by apoptosis, antibody-dependent cell-mediated cytotoxicity (ADCC), and complement-dependent cytotoxicity (CDC); and
   (ii) at least one lenalidomide compound, preferably lenalidomide; and, optionally
   (iii) a dexamethasone compound, preferably dexamethasone; and, optionally
   (iv) an anti-coagulation agent.
30. The combination of item 29, wherein the combination is for sequential use in the treatment of a hematological malignancy, preferably multiple myeloma.

The following examples are intended to illustrate but not to limit the invention.

### EXAMPLES

hu38SB19 was provided in solution at 5 mg/ml, stored at 4°C. It was diluted into sterile saline in preparation for dosing, stored at 4°C and used within 10 days of dilution.

Lenolidomide was obtained from AK Scientific Inc. (Mountain View, CA) and prepared as a suspension in 1% (w/v) carboxymethyl cellulose (Sigma). Preparation was made using a mortar and pestle to generate a slurry suspension in the vehicle, diluted to the appropriate concentration, and used for dosing by oral gavage.

### Example 1: Effect of the administration of both anti-CD38 antibody and lenalidomide in a mice model of MM

These studies under this Example were done under approval of the UCSF IACUC.

The subcutaneous multiple myeloma (MM) xenograft mouse models were established using H929 and RPMI8226 cell lines. Specifically, 5-6 week old female Balb/c Scid mice were obtained from Jackson Lab. Mice were housed for 7-10 days prior to implantation. Mice were housed in a dedicated room in the UCSF Mt Zion Animal Barrier Facility. NCI-H929 and RPMI-8226 cells were obtained from the German Collection of Microorganisms and Cell Cultures, DSMZ, (Deutsche Sammlung von Mikroorganismen und Zellkulturen), and grown in sterile suspension culture in T225 flasks as follows: NCI-H929: RPMI1640 + 20% FBS + 4 mM L-glutamine + 1 mM sodium pyruvate + 50 µM mercaptoethanol. RPMI-8226: RPMI1640 + 10% FBS + 4 mM L-glutamine.

At the time of implantation, mice were shaved on the right flank and shoulder region and anesthetized with ip avertin. MM cells suspended in serum free RPMI 1640 media diluted 1:1 with Matrigel (BD) at a concentration of 1x10⁸ cells per ml were injected sc into the right flank in 100 µL volume (1x10⁷ cells) using a 1 ml syringe and 25 g needle. Mice were monitored twice weekly for the appearance of tumors and once tumors were visible, measurements were collected twice weekly for body weight and tumor volume. Electronic balance and calipers were used and data was collected directly into a study management program (Study Director). When the mean tumor volume reached about 150-200 mm³, the mice were distributed into treatment groups of 8-10 mice per groups and dosing was begun.

The dosing schedule was hu38SB19 was 2x/wk x 2 wk (iv lateral tail vein) and lenalidomide was qdx7 x 3 wk (po) (orally, one dose per day, 7 days a week, for 21 days). Dose levels for use in combination studies are as follows:

| **Cell Type** | **Lenalidomide** | **hu38SB19** |
|---|---|---|
| H929 | 1 mpk | 0.5 mpk |
| RPMI8226 | 15 mpk | 15 mpk |
| mpk = mg per kg body weight | | |

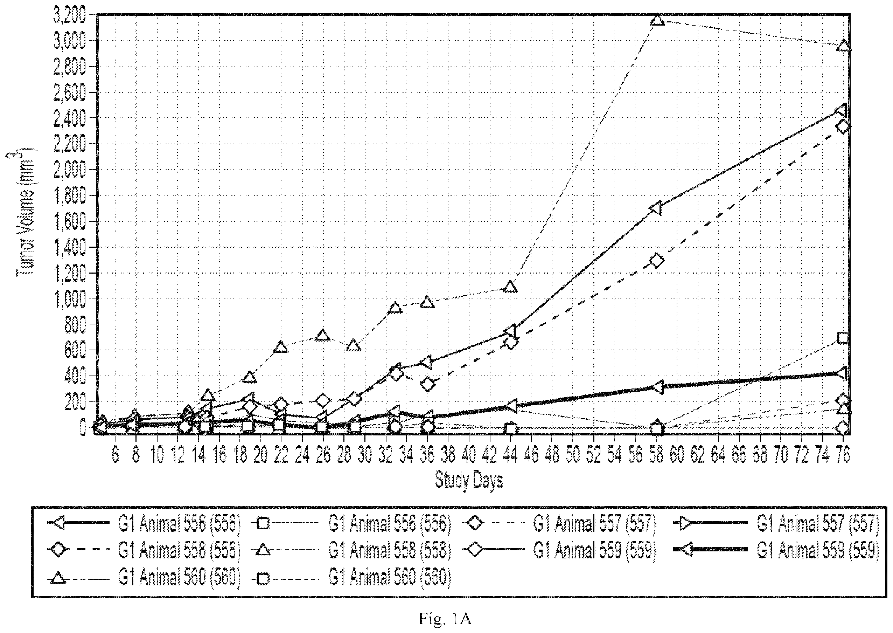

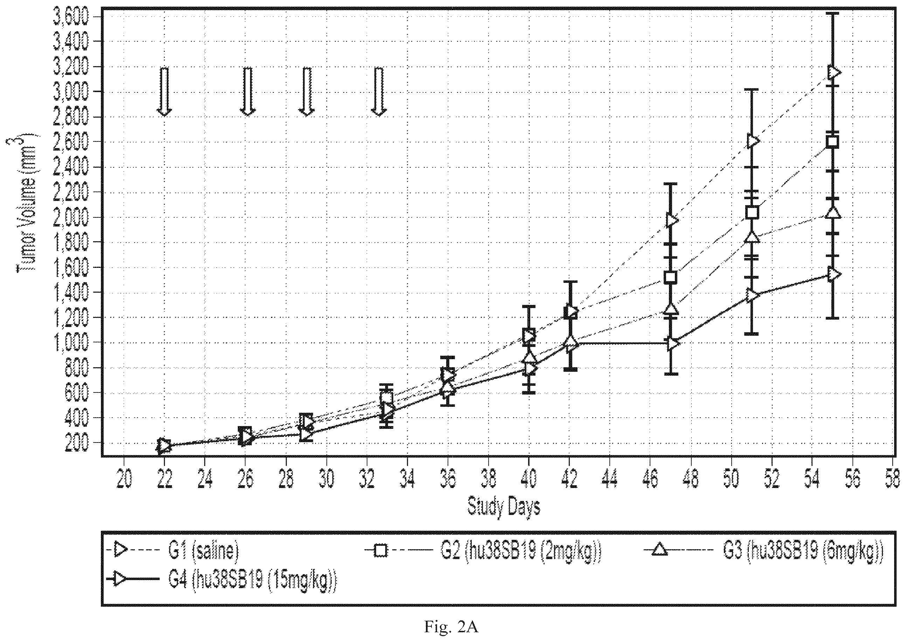

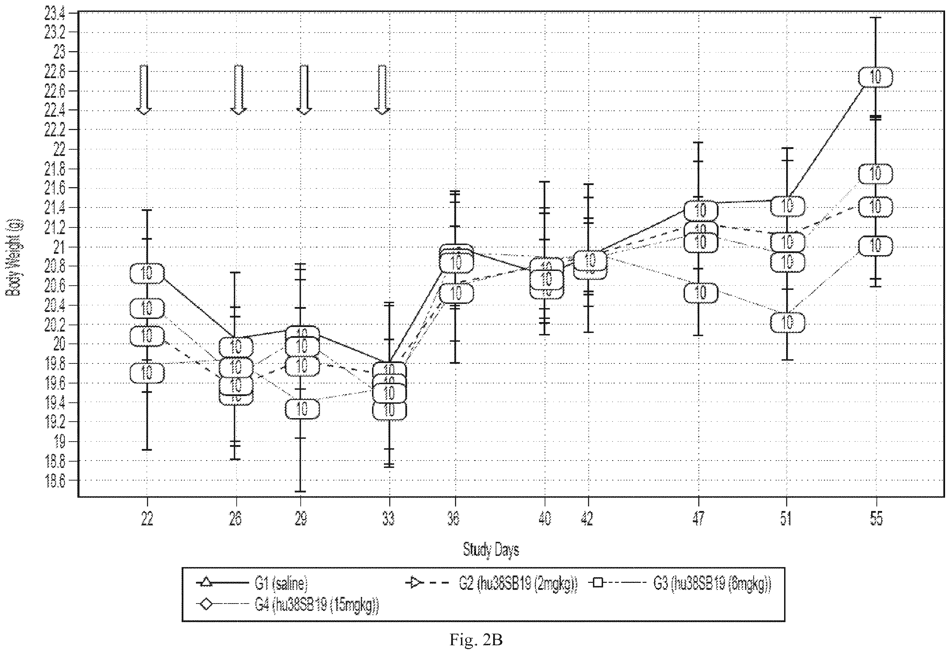

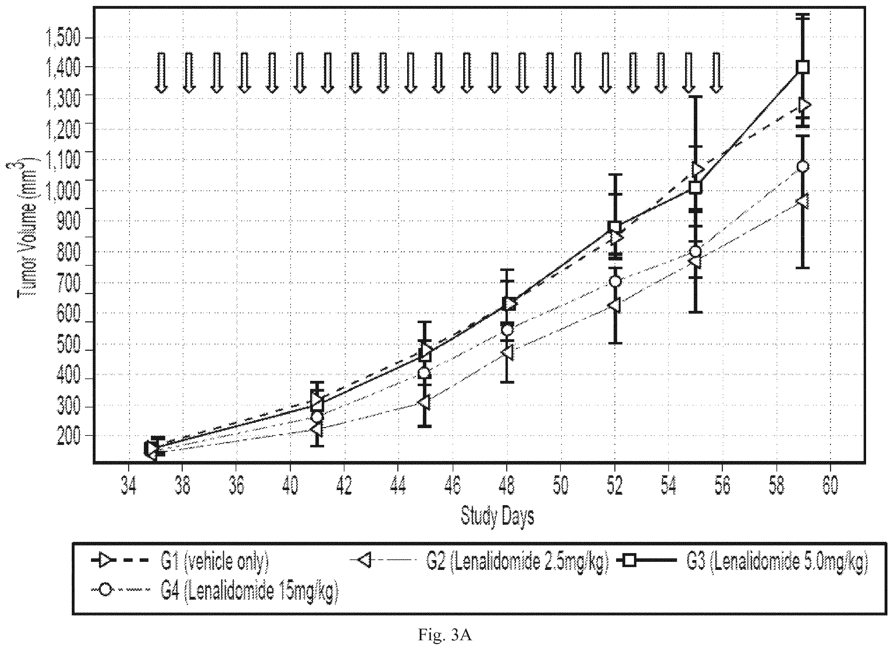

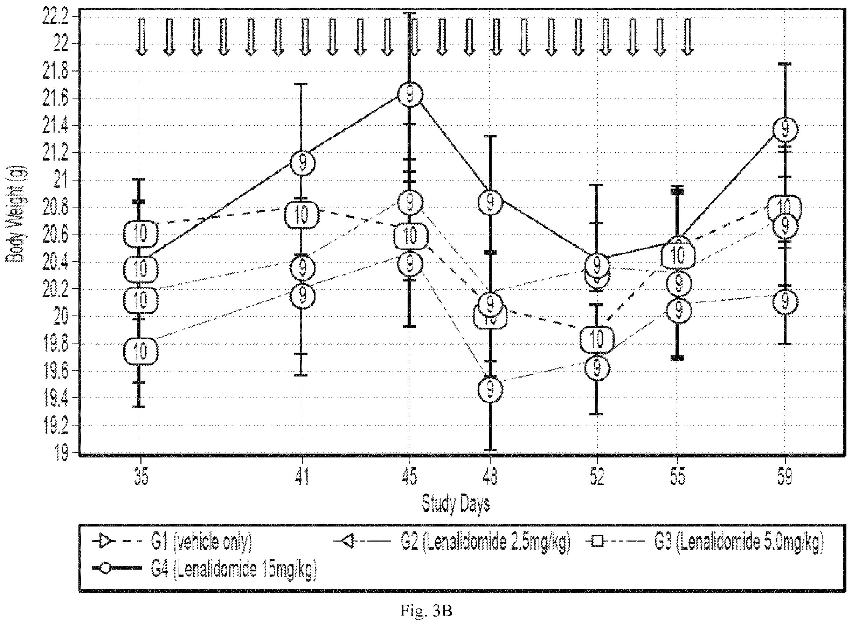

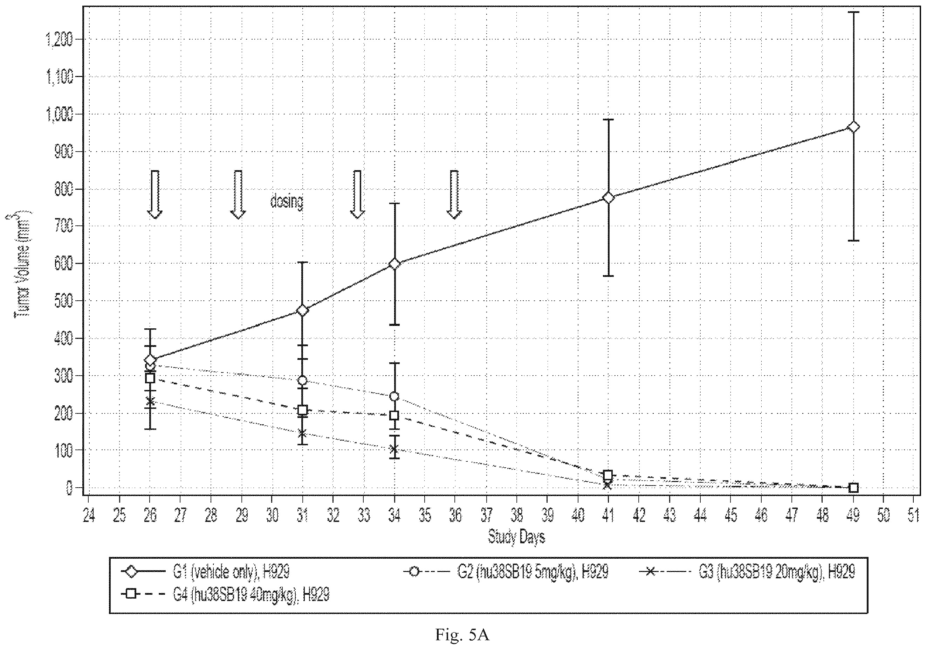

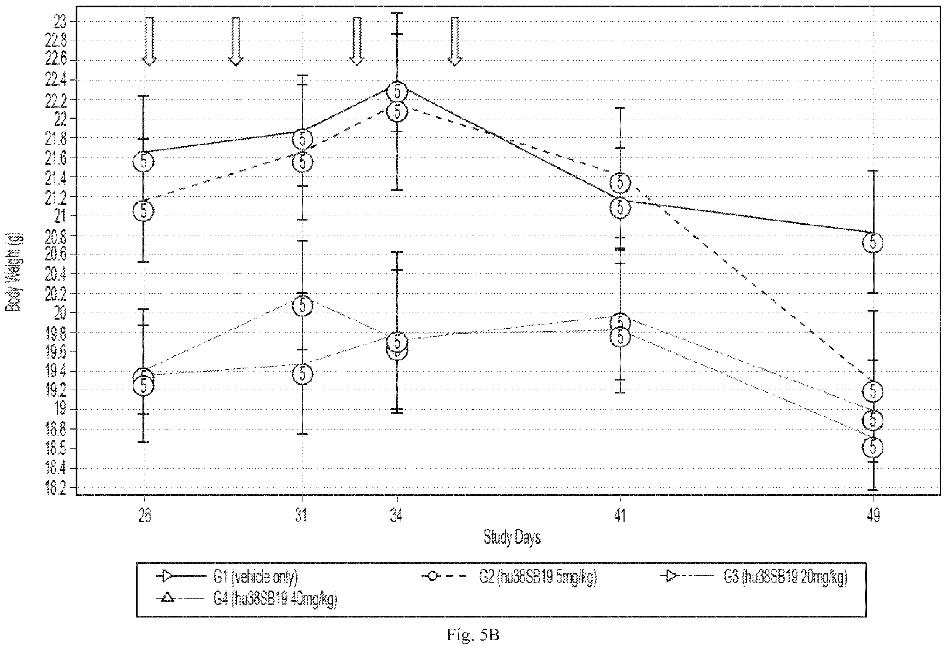

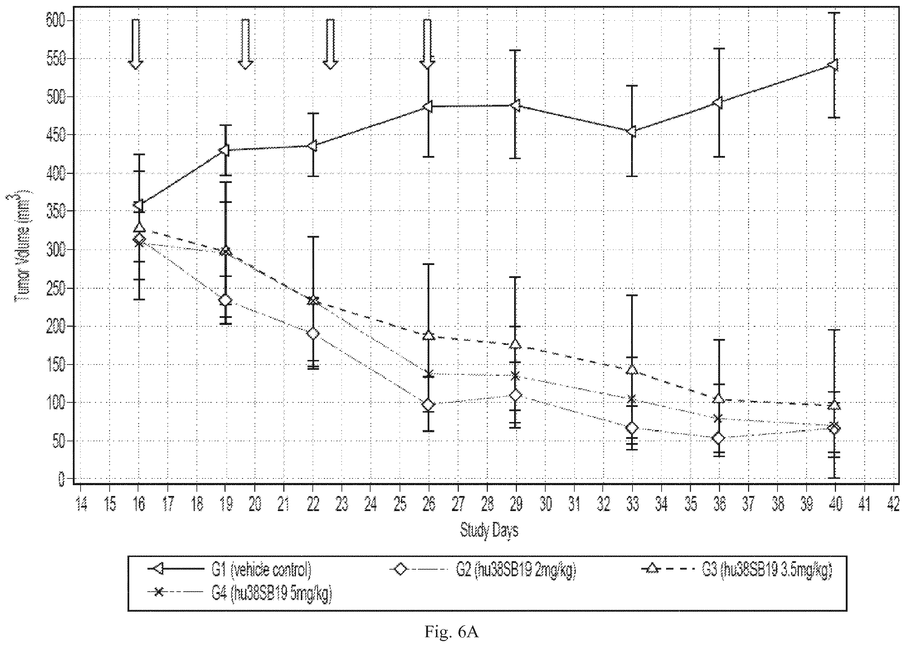

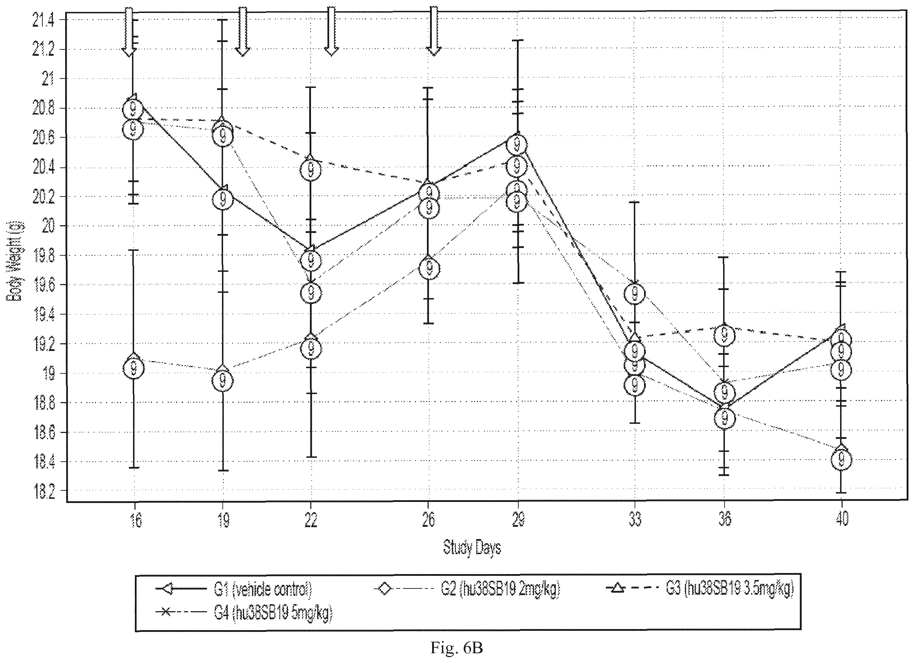

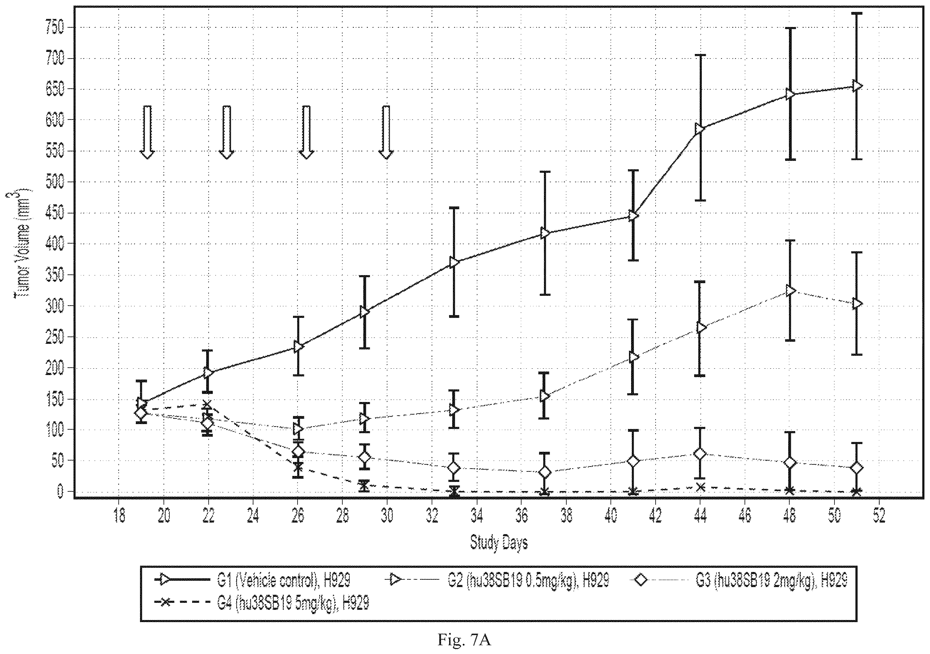

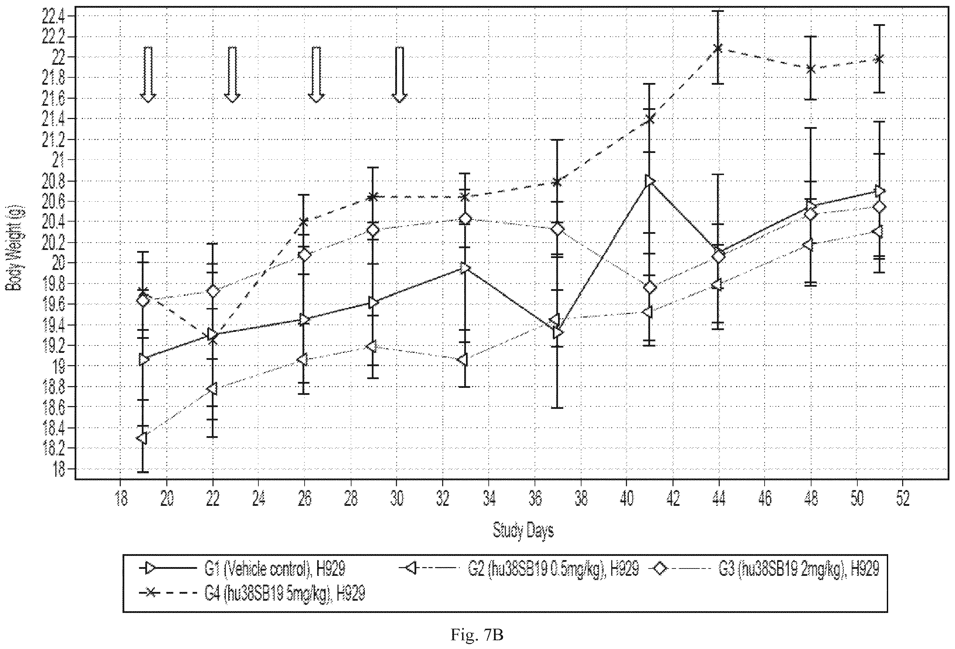

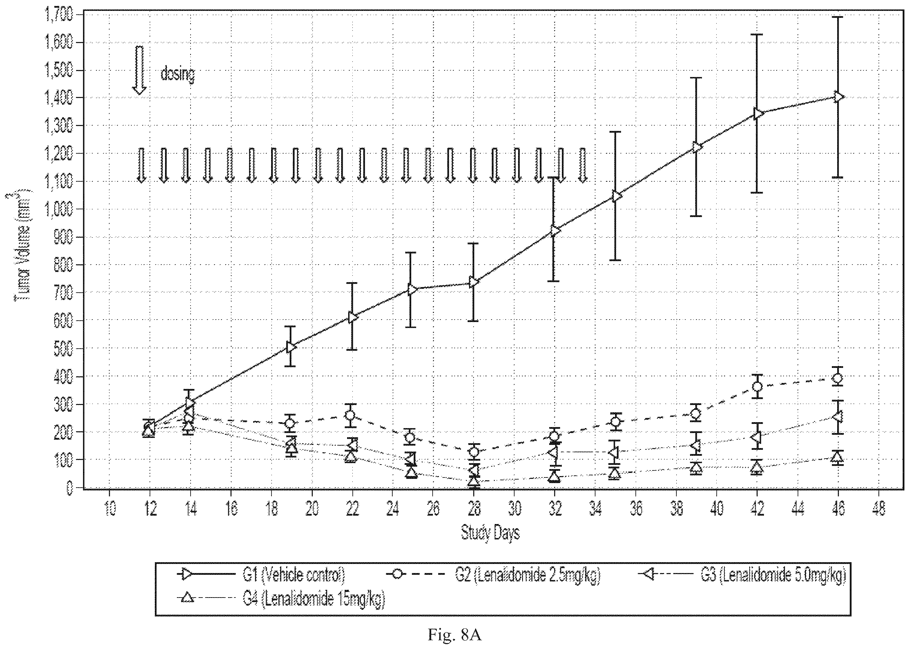

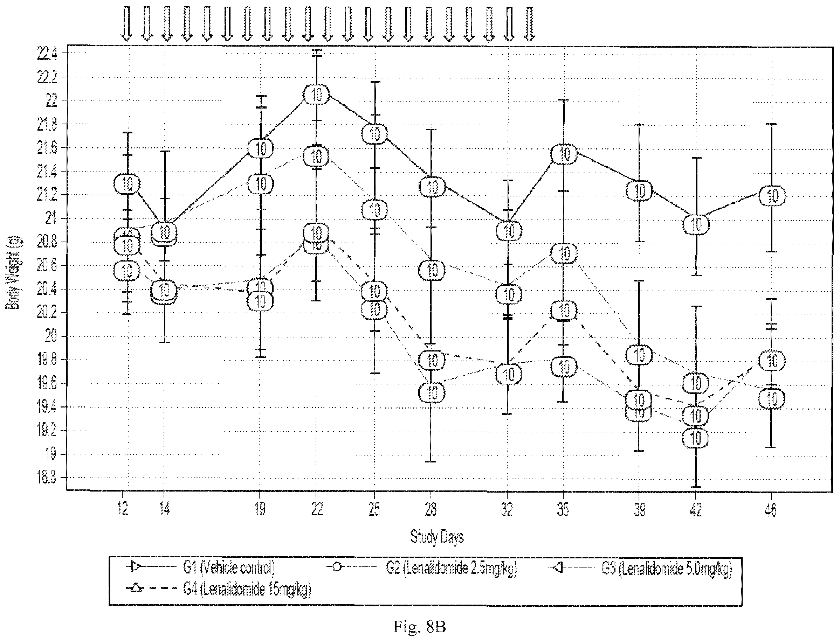

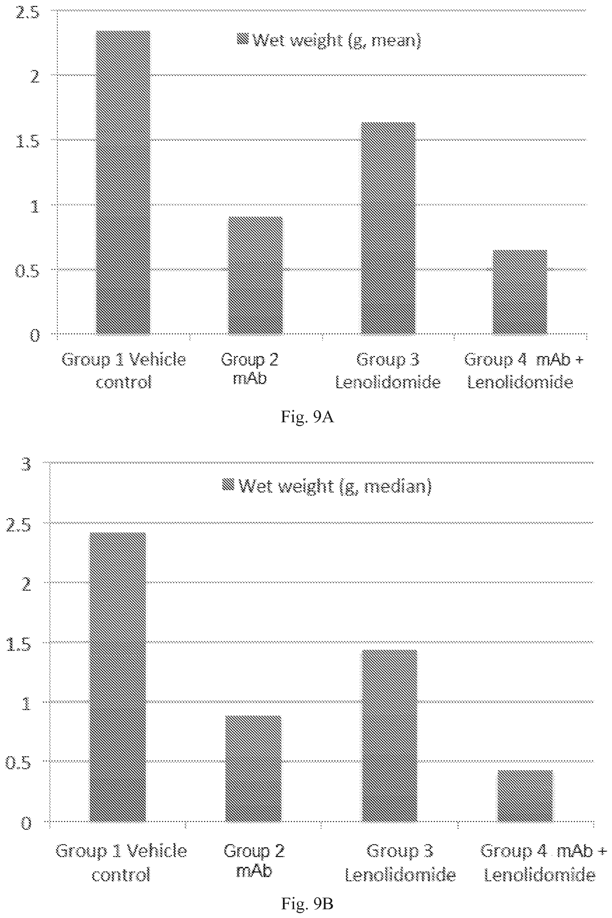

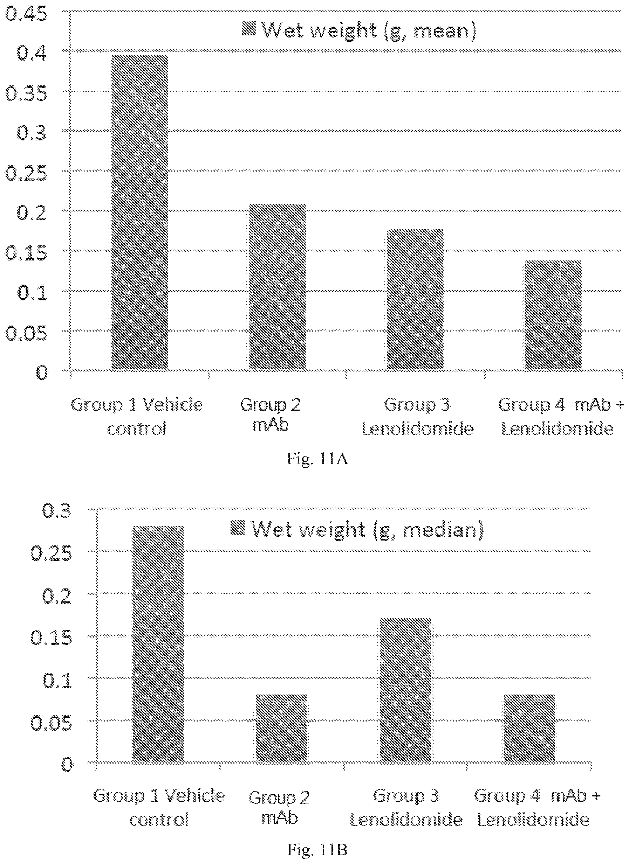

Data were collected using electronic balance and calipers using a study management application called StudyLog (Study Director). Graphs are taken directly from the application. The experimental results are provided in Figures 1A-11B.

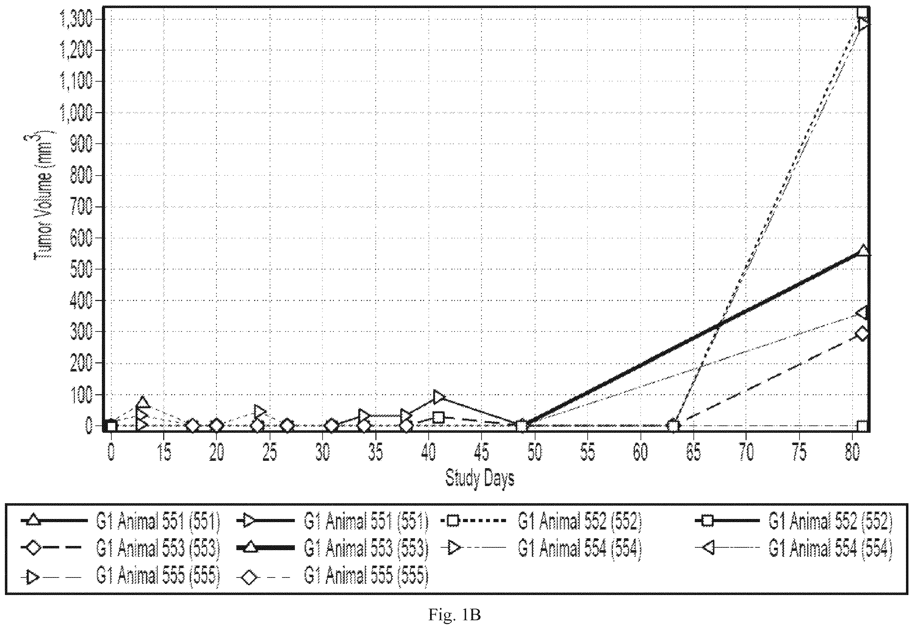

Based on the single agent results of hu38SB19 and lenalidomide in RPMI-8226 and NCI-H929 mutiple myeloma xenograft models, NCI-H929 appears to be a more sensitive model to both agents while RPMI-8226 seems to be more resistant to the treatments even at the highest doses tested (Figures 1-3, 5-8). Therefore in the combination studies, a suboptimal dose for each agent was chosen to evaluate the activity of the combination treatment (lenalidomide + hu38SB19) in the NCI-H929 model while higher doses of lenalidomide and hu38SB19 were tested in the RPMI-8226 model.

Antitumor activity was determined according to NCI standards based on the ratio of the median tumor volume change of the treated / median tumor volume change of the control x 100 (%ΔT/ΔC). Low numerical values for ΔT/ΔC describe stronger anti-tumor activity. Anti-tumor activity is defined as ΔT/ΔC ≤ 40% at minimum. ΔT/ΔC <10% is considered high anti-tumor activity.

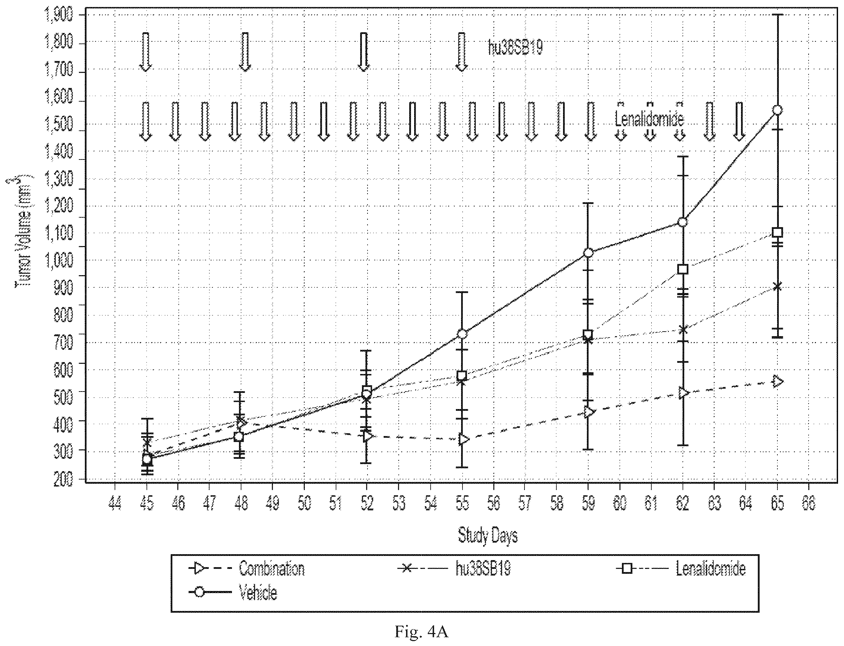

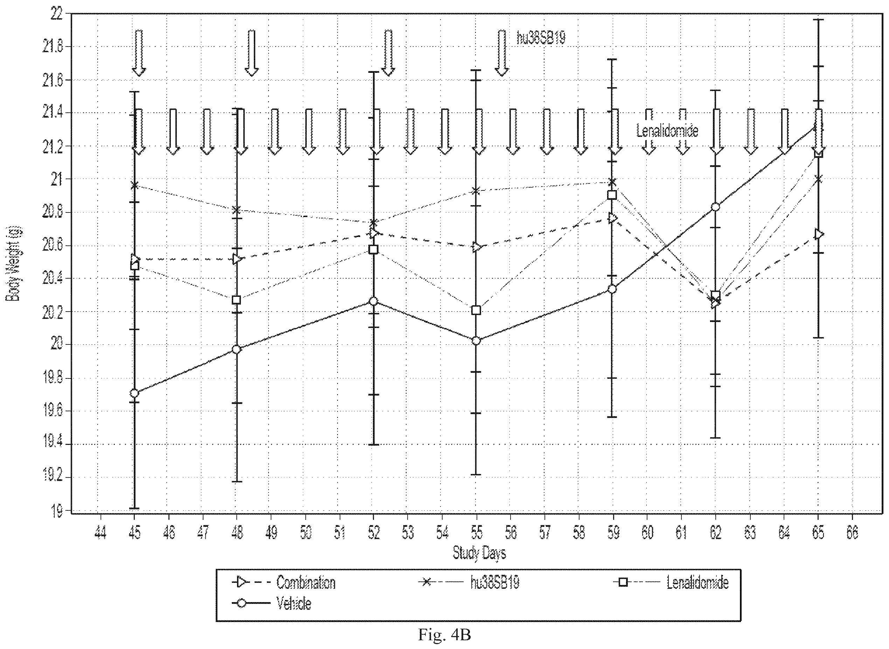

In the RPMI-8226 model, hu38SB19 alone at 15 mg/kg/injection (twice a week for 2 weeks) was inactive with a %ΔT/ΔC of 44%. Treatment with lenalidomide alone at 15 mg/kg/day (dosed daily for three weeks) was inactive (61%ΔT/ΔC). The combination of hu38SB19 (15 mg/kg/injection) and lenalidomide (15 mg/kg/day) had higher activity with %T/C of 13% (Figure 4). The results are summarized in Table 1.

| **TABLE 1** | | | | |
|---|---|---|---|---|
| **Anti-tumor efficacy of hu38SB19 in combination with lenalidomide against RPMI-8226 multiple myeloma model** | | | | |
| **Agent** | **Dose in mg/kg (total dose)** | **Schedule ofAdministration IV or PO route** | **%ΔT/ΔC(D69)** | **Activity** |
| PBS | - | 2x/wk x 2 wk (IV) | | |
| hu38SB19 | 15 (60) | 2x/wk x 2 wk (IV) | 44 | Inactive |
| Lenalidomide | 15 (315) | QD x 21 d (PO) | 61 | Inactive |
| hu38SB19 + Lenalidomide | 15 (60) + 15 (315) | 2x/wk x 2 wk (IV) + QD x 21 d (PO) | 13 | Active |
| %ΔT/ΔC Median tumor volume change of the treated / Median tumor volume change of the control x 100, IV=intravenous, PO=oral, d=days, wk=week, QD=once daily, PBS: phosphate buffered saline | | | | |

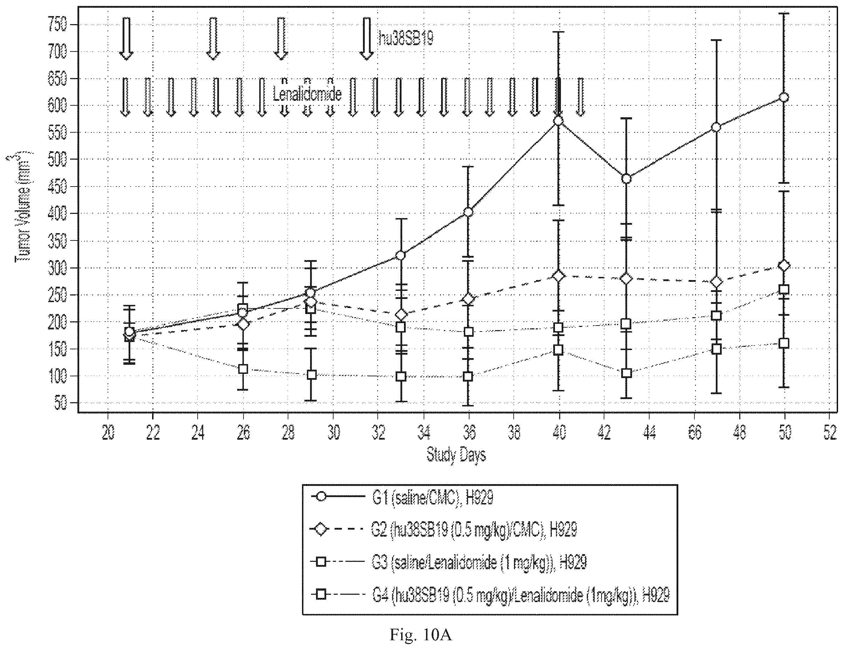

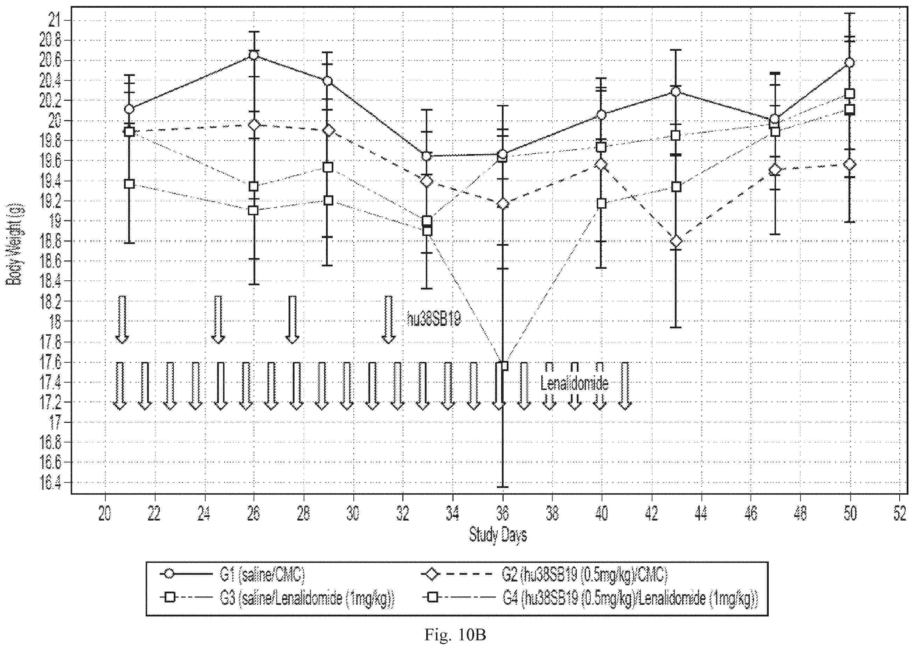

In the NCI-H929 model, hu38SB19 alone at 0.5 mg/kg/injection (twice a week for 2 weeks) was active with a %ΔT/ΔC of 10%. Treatment with lenalidomide alone at 1 mg/kg/day (dosed daily for three weeks) was active (20%ΔTΔ/C). The combination of hu38SB19 (0.5 mg/kg/injection) and lenalidomide (1 mg/kg/day) had higher activity (tumor regression) with %ΔT/ΔC of -8% (Figure 10). The results are summarized in Table 2.

| **TABLE 2** | | | | |
|---|---|---|---|---|
| **Anti-tumor efficacy of hu38SB19 in combination with lenalidomide against NCl-H929 multiple myeloma model** | | | | |
| **Agent** | **Dose in mg/kg (total dose)** | **Schedule of Administration IV or PO route** | **%ΔT/ΔC (D69)** | **Activity** |
| PBS | - | 2x/wk x 2 wk (IV) | | |
| hu38SB19 | 0.5 (2) | 2x/wk x 2 wk (IV) | 10 | Active |
| Lenalidomide | 1 (21) | QD x 21 d (PO) | 20 | Active |
| hu38SB19 + Lenalidomide | 0.5 (2) + 1 (21) | 2x/wk x 2 wk (IV) + QD x 21 d (PO) | -8 | Highly Active |
| %ΔT/ΔC Median tumor volume change of the treated / Median tumor volume change of the control x 100, IV=intravenous, PO=oral, d=days, wk=week, QD=once daily, PBS: phosphate buffered saline | | | | |

In both models, the combination treatment inhibited tumor growth to a much greater extent than a single agent alone, indicating the combination of hu38SB19 and lenalidomide blocked tumor cell growth through potential synergistic mechanisms. Although the molecular mechanisms of action of lenalidomide is still unknown, it is generally believed that lenalidomide enhances natural killer cell activity which is important for antibody dependent cellular cytotoxicity (ADCC) and directly induces apoptosis in tumor cells. Hu38SB19 has demonstrated potent ADCC and direct apoptosis induction activity on tumor cells and these activities are further enhanced by lenalidomide as evidenced by the experiments herein.

It has been reported that some CD38 antibodies such as Daratumumab is able to induce apoptosis only after cross-linking with a secondary antibody without much direct effect by itself. However, in preclinical studies, hu38SB19 demonstrated potent direct pro-apoptotic activity on tumor cells without cross-linking. Thus, this unique property of hu38SB19 may also lead to greater tumor cell killing when in combination with lenalidomide compared to other CD38 antibodies combined with lenalidomide.

### Example 2: Effect of the administration of both anti-CD38 antibody and lenalidomide in humans

A Phase 1b study for evaluating the effects of a treatment with hu38SB19 combined with lenalidomide and low dose dexamethasone in patients with relapsed or refractory multiple myeloma is performed as described below.

The main goals of the Phase 1b study include:
- To determine the efficacy and the maximum tolerated dose;
- To evaluate the safety, including immunogenicity, of hu38SB19 in combination with lenalidomide in relapse or refractory multiple myeloma. The severity, frequency and incidence of all toxicities is assessed;
- To evaluate the pharmacokinetics (PK) of hu38SB19 when administered in combination with lenalidomide and the PK of lenalidomide in combination with HU38SB19 and dexamethasone.
- To assess the relationship between clinical (adverse event and/or tumor response) effects and pharmacologic parameters (PK/pharmacodynamics), and/or biologic (correlative laboratory) results;
- Estimate the activity (response rate) using International Myeloma Working Group defined response criteria of hu38SB19 plus lenalidomide and dexamethasone; and
- To describe overall survival, progression free survival (PFS) and time to disease progression in patients treated with this combination.

About 20 to 40 patients may be selected based on the following criteria: The patients are male or female, but must be diagnosed with multiple myeloma and be aged of at least 18 years. For each patient, there is a documentation of at least 2 prior therapies (induction therapy is considered one prior therapy). There is no maximum number of prior regimens and prior bone marrow transplant is acceptable. There is a confirmed evidence of disease progression from immediately prior MM therapy or refractory to the immediately prior therapy. Patients may have received prior immunomodulatory drugs (IMiDs) (e.g., lenalidomide or thalidomide). Patients are with measurable disease. Patients are with a Karnofsky ≥60% performance status. Females of childbearing potential are included provided they have a negative serum or urine pregnancy test with a sensitivity of at least 50 mIU/mL within 10 to 4 days and again within 24 hours prior to prescribing lenalidomide for Cycle 1 (prescriptions must be filled within 7 days as required by RevAssist®) and must either commit to continued abstinence from heterosexual intercourse or begin two acceptable methods of birth control, one highly effective method and one additional effective method at the same time, at least 28 days before she starts taking lenalidomide. Females of childbearing potential must also agree to ongoing pregnancy testing. Ability to understand the purpose and risks of the study and provide signed and dated informed consent and authorization to use protected health information (in accordance with national and local subject privacy regulations). The patient must be able to take aspirin daily as prophylactic anti-coagulation therapy (patients intolerant to aspirin may use warfarin, low molecular weight heparin or equivalent anti-platelet therapy).

In addition, patients meeting at least one of the following criteria are excluded:
- Diagnosed or treated for another malignancy within 3 years prior to enrollment, with the exception of complete resection of basal cell carcinoma or squamous cell carcinoma of the skin, an in situ malignancy, or low risk prostate cancer after curative therapy;
- Prior anti-cancer therapy (chemotherapy, targeted agents, radiotherapy, and immunotherapy) within 21 days except for alkylating agents (e.g., melphalan) where 28 days will be required or participated in another clinical trial during the past 30 days;
- History of significant cardiovascular disease within the past 6 months, unless the disease is well-controlled. Significant cardiac diseases includes second/third degree heart block; significant ischemic heart disease (eg, angina); QTc interval >450 msec at baseline (read by local cardiologist); poorly controlled hypertension; congestive heart failure of New York Heart Association (NYHA) Class II (slight limitation of physical activity; comfortable at rest, but ordinary physical activity results in fatigue, palpitation, or dyspnea) or worse; left-ventricular ejection fraction (LVEF) <50%;
- Prior peripheral stem cell transplant within 12 weeks of the first dose of study treatment;
- Daily requirement for corticosteroids (>10 mg/kg prednisone qd) (except for inhalation corticosteroids);
- Evidence of mucosal or internal bleeding;
- Prior radiation therapy or major surgical procedure within 4 weeks of the first dose of study treatment;
- Known active infection requiring parenteral or oral anti-infective treatment;
- Serious psychiatric illness, active alcoholism, or drug addiction that may hinder or confuse follow-up evaluation;
- Any medical conditions that, in the Investigator's opinion, would impose excessive risk to the patient. Examples of such conditions include any pre-existing kidney disease (acute or chronic, unless renal insufficiency is felt to be secondary to MM, hypertension, active seizure disorder or pulmonary diseases that would impose excessive risk to the patient;
- Hypersensitivity to any of the components of study therapy that is not amenable to premedication with steroids and H2 blockers;
- Known human immunodeficiency virus (HIV) or active hepatitis B or C viral infection;
- Neuropathy ≥ Grade 3 or painful neuropathy ≥ Grade 2 (National Cancer Institute Common Terminology Criteria for Adverse Events [NCI CTCAE] v 4.0);
- Gastro-intestinal abnormalities, including bowel obstruction, inability to take oral medication, requirement for intravenous (IV) alimentation, active peptic ulcer or prior surgical procedures or bowel resection affecting absorption; and
- Pregnancy.

The patients are treated with hu38SB19 combined with lenalidomide and dexamethasone. hu38SB19 is administered intravenously as a solution. Lenalidomide is administered orally as capsules. Dexamethasone is administered orally as tablets. The study duration for an individual patient includes a screening period for inclusion of up to 21 days, and at least 4 weeks of treatment in the absence of severe adverse reaction, dose limiting toxicity or disease progression plus up to 60 days post-treatment follow up. The total duration of the study may be up to one year.

The following parameters are measured during and/or at the end of the study:
- Number of patients with adverse events when treated with hu38SB19 in combination with Lenalidomide
- Assessment of partial response, complete response, progression free survival, and survival;
- Assessment of the following PK parameters: area under curve (AUC), maximum concentration (Cmax) and plasma half-life (T 1/2)
- Number of CD38 receptors occupied by hu38SB19; and
- Number of anti-SAR antibodies in response to hu38SB19.

### Example 3: Efficacy of anti-CD38 antibody in in vivo tumor models of multiple myeloma as a single-agent or in combination with and lenalidomide in humans the standard-of-care immunomodulatory targeting agent, Lenalidomide.

### A. Materials and Methods

CD38 Density: CD38 density was determined using anti-CD38-PE Quantibrite (BD Biosciences; Cat.342371) per the manufacturer's recommended protocols.

Reagents & Compounds: hu38SB19 was provided by Sanofi Oncology in solution at 5 mg/ml and stored at 4° C. hu38SB19 was diluted into sterile saline in preparation for dosing and used within 10 days of dilution. hu38SB19 was administered twice weekly x 2 wk IV. Lenolidomide (TC27682) was obtained from AK Scientific Inc. (Mountain View, CA) and prepared as a suspension in 1% (w/v) carboxymethyl cellulose (Sigma). Preparation was made using a mortar and pestle to generate a slurry suspension in the vehicle, diluted to the appropriate concentration, and used for dosing by oral gavage. Lenalidomide was administered qdx7 x 3 wk PO.

Test Animals: 5-6 week old female Balb/c Scid mice were obtained from Jackson Lab. Mice were housed for 7-10 days prior to implantation of multiple myeloma (MM) cell lines. Mice were housed in a dedicated room in the UCSF Mt. Zion Animal Barrier Facility.

Cell culture: RPMI-8226 cells were obtained from the German Collection of Microorganisms and Cell Cultures, DSMZ, (Deutsche Sammlung von Mikroorganismen und Zellkulturen), and grown in sterile suspension culture in T225 flasks. RPMI-8226 were cultured in RPMI1640 + 10% FBS + 4 mM L-glutamine.

Xenograft Model: At the time of implantation, mice were shaved on the right flank and shoulder. MM cells were suspended in serum free RPMI 1640 media diluted 1:1 with Matrigel (BD) at a concentration of 1x10⁸ cells per ml were injected sc into the right flank in 100 ul volume (1x10⁷ cells) using a 1 ml syringe and 25 g needle. Mice were monitored twice weekly for the appearance of tumors and once tumors were visible, measurements were collected twice weekly for body weight and tumor volume. Electronic balance and calipers were used and data was collected directly into a study management program (Study Director). When the mean tumor volume reached approximately 150-200 mm³, mice were distributed into treatment groups of 8-10 mice per group and dosing was initiated.

### B. Summary and Conclusions

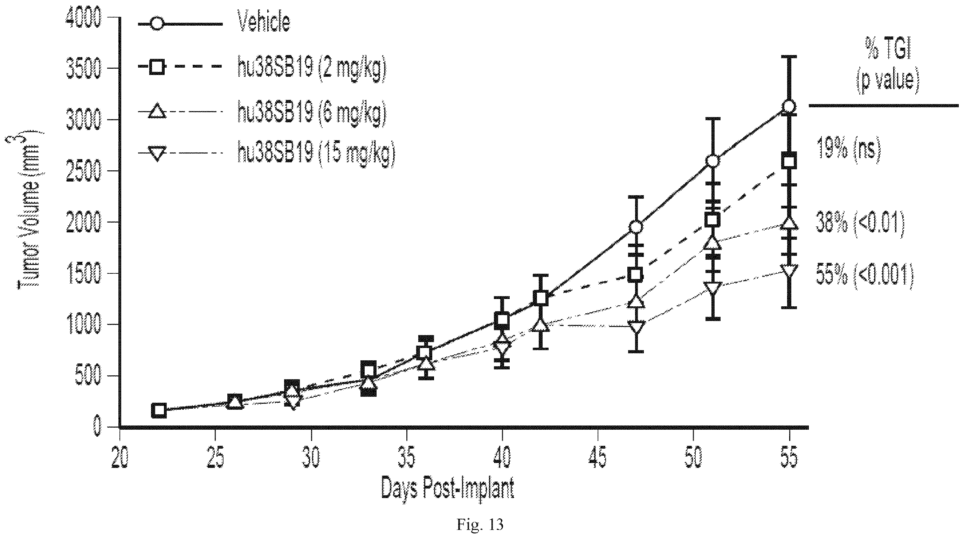

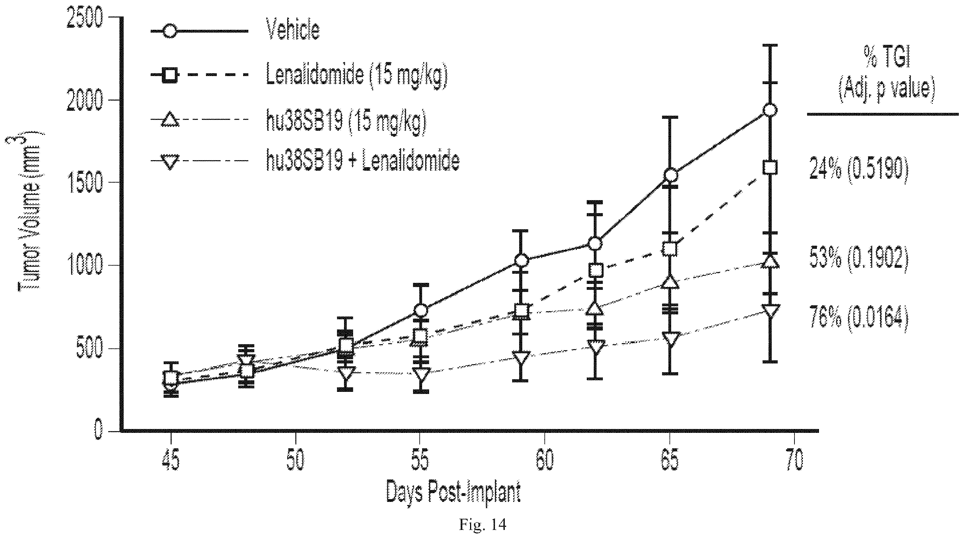

hu38SB19 is a humanized anti-CD38 antibody whose anti-myeloma effects incorporate mechanisms of ADCC, CDC, and direct apoptosis. Figure 21 shows the cell surface density of CD38 in multiple myeloma cell lines. See Kim D, Park CY, Medeiros BC, Weissman IL. CD19-CD45 low/- CD38 high/CD138+ plasma cells enrich for human tumorigenic myeloma cells. Leukemia. 2012 Dec, 26(12):2530-7. CD38-positive multiple myeloma plasma cells demonstrate variable CD38 cell surface densities. All cell lines, with the exception of XG-6, are reported as CD38-positive. See Bataille R, Jégo G, Robillard N, et al. The phenotype of normal, reactive and malignant plasma cells. Identification of "many and multiple myelomas" and of new targets for myeloma therapy. Haematologica. 2006 Sept, 91(9):1234-40. Binding of hu38SB19 to CD38 also impinges on the ADPRC enzymatic activity of CD38. *In vivo,* hu38SB19 demonstrates potent anti-tumor effects in multiple myeloma xenografts, a disease largely characterized by neoplastic plasma cells expressing CD38. Figure 13 shows that single-agent administration of hu38SB19 results in dose-dependent inhibition of tumor growth in an RPMI-8226 hind-flank model. The magnitude and significance of tumor growth inhibition at the end of the study increased with increased doses of hu38SB19. Figure 14 shows that a combined regimen of hu38SB19 and Lenalidomide results in significant tumor growth inhibition in an RPMI-8226 xenograft model that is not robustly sensitive to single-agent therapy with Lenalidomide. These data demonstrate that single-agent hu38SB19 inhibits growth of RPMI-8226 tumors and combines with sub-efficacious doses of Lenalidomide to produce significant inhibition of tumor growth. Taken together, these data support further evaluation of hu38SB19, both as a single-agent and in combination with standard-of-care treatment regimens, as a potential therapy for the treatment of multiple myeloma.

To the extent necessary to understand or complete the disclosure of the present invention, all publications, patents, and patent applications mentioned herein are expressly incorporated by reference therein to the same extent as though each were individually so incorporated.

Having thus described exemplary embodiments of the present invention, it should be noted by those skilled in the art that the within disclosures are exemplary only and that various other alternatives, adaptations, and modifications may be made within the scope of the present invention. Accordingly, the present invention is not limited to the specific embodiments as illustrated herein, but is only limited by the following claims.

## Claims

1. A method of treating a cancer in a subject which comprises administering one or more anti-CD38 antibodies and one or more lenalidomide compounds to the subject.

2. The method of claim 1, wherein the cancer is a hematological malignancy, optionally wherein the hematological malignancy is multiple myeloma, optionally, wherein the multiple myeloma is a relapsed multiple myeloma or a refractory multiple myeloma.

3. The method of claim 1, wherein the one or more lenalidomide compounds is lenalidomide.

4. The method of claim 1, wherein the one or more anti-CD38 antibodies are administered in an effective amount, wherein the one or more lenalidomide compounds are administered in a therapeutically effective amount, and/or wherein the one or more anti-CD38 antibodies and the one or more lenalidomide compounds are administered in a synergistic amount.

5. The method of any one of claims 1 to 4, wherein at least one of the one or more anti-CD38 antibodies is capable of killing a CD38⁺ cell by apoptosis, antibody-dependent cell-mediated cytotoxicity (ADCC), and complement-dependent cytotoxicity (CDC).

6. The method of any one of claims 1 to 5, wherein at least one of the one or more anti-CD38 antibodies is selected from the group consisting of:
a) an antibody comprising a heavy chain comprising three sequential CDRs having amino acid sequences consisting of SEQ ID NOs: 13, 15 and either SEQ ID NO: 14 or SEQ ID NO: 81, and a light chain comprising three sequential CDRs having amino acid sequences consisting of SEQ ID NOs: 16, 17 and 18;
b) an antibody comprising a heavy chain comprising three sequential CDRs having amino acid sequences consisting of SEQ ID NOs: 25, 26 and 27, and a light chain comprising three sequential CDRs having amino acid sequences consisting of SEQ ID NOs: 28, 29 and 30;
c) an antibody comprising a heavy chain comprising three sequential CDRs having amino acid sequences consisting of SEQ ID NOs: 1, 2 and 3, and a light chain comprising three sequential CDRs having amino acid sequences consisting of SEQ ID NOs: 4, 5 and 6;
d) an antibody comprising a heavy chain comprising three sequential CDRs having amino acid sequences consisting of SEQ ID NOs: 7, 8 and 9, and a light chain comprising three sequential CDRs having amino acid sequences consisting of SEQ ID NOs: 10, 11 and 12;
e) an antibody comprising a heavy chain comprising three sequential CDRs having amino acid sequences consisting of SEQ ID NOs: 19, 20 and 21, and a light chain comprising three sequential CDRs having amino acid sequences consisting of SEQ ID NOs: 22, 23 and 24; and
f) an antibody comprising a heavy chain comprising three sequential CDRs having amino acid sequences consisting of SEQ ID NOs: 31, 32 and 33, and a light chain comprising three sequential CDRs having amino acid sequences consisting of SEQ ID NOs: 34, 35 and 36.

7. The method of claim 6, wherein said antibody comprises
a heavy chain having a V_{H} variable region represented by SEQ ID NO: 66, and a light chain having a V_{L} variable region represented by either SEQ ID NO: 62 or SEQ ID NO: 64; or
a heavy chain having a V_{H} variable region represented by SEQ ID NO: 72, and a light chain having a V_{L} variable region represented by either SEQ ID NO: 68 or SEQ ID NO: 70.

8. The method of any one of claims 1 to 7, wherein the one or more anti-CD38 antibodies are administered intravenously and/or wherein the one or more lenalidomide compounds are administered orally.

9. The method of any one of claims 1 to 8, wherein the one or more anti-CD38 antibodies and the one or more lenalidomide compounds are administered sequentially.

10. The method of any one of claims 1 to 9, and further comprising administering a dexamethasone compound, to the subject, optionally wherein the dexamethasone compound is dexamethasone, optionally wherein the dexamethasone compound is administered orally, optionally wherein the dexamethasone is administered at a low dose.

11. The method of any one of claims 1 to 10, and further comprising administering an anti-coagulation agent to the subject, optionally wherein the anti-coagulation agent is selected from the group consisting of aspirin, warfarin, and low molecular weight heparin.

12. A kit comprising
at least one anti-CD38 antibody capable of killing a CD38⁺ cell by apoptosis, antibody-dependent cell-mediated cytotoxicity (ADCC), and complement-dependent cytotoxicity (CDC), packaged together with
a label having one or more messages that the at least one anti-CD38 antibody shall be administered in combination with lenalidomide, and optionally with dexamethasone and/or an anti-coagulation agent.

13. A combination of:
(i) at least one anti-CD38 antibody, preferably the antibody is capable of killing a CD38+ cell by apoptosis, antibody-dependent cell-mediated cytotoxicity (ADCC), and complement-dependent cytotoxicity (CDC); and
(ii) at least one lenalidomide compound, preferably lenalidomide; and, optionally
(iii) a dexamethasone compound, preferably dexamethasone; and, optionally
(iv) an anti-coagulation agent.


SEQUENCE LISTING

<110>  SANOFI
       THE REGENTS OF THE UNIVERSITY OF CALIFORNIA

<120>  COMPOSITIONS COMPRISING ANTI-CD38 ANTIBODIES AND LENALIDOMIDE

<130>  BET 19M0114

<150>  US61/734,524
<151>  2012-12-07

<150>  US61/769,247
<151>  2013-02-26

<150>  US61/808,372
<151>  2013-04-04

<150>  EP13861470.6
<151>  2013-12-06

<160>  81

<170>  PatentIn version 3.5

<210>  1
<211>  5
<212>  PRT
<213>  Mus sp.

<400>  1

Ser Tyr Gly Met Asn
1               5

<210>  2
<211>  17
<212>  PRT
<213>  Mus sp.

<400>  2

Trp Ile Asn Thr Tyr Thr Gly Glu Pro Thr Tyr Ala Asp Asp Phe Lys
1               5                   10                  15

Gly

<210>  3
<211>  5
<212>  PRT
<213>  Mus sp.

<400>  3

Arg Gly Phe Ala Tyr
1               5

<210>  4

<211>  15
<212>  PRT
<213>  Mus sp.

<400>  4

Arg Ala Ser Glu Ser Val Glu Ile Tyr Gly Asn Gly Phe Met Asn
1               5                   10                  15


<210>  5
<211>  7
<212>  PRT
<213>  Mus sp.

<400>  5

Arg Ala Ser Asn Leu Glu Ser
1               5


<210>  6
<211>  9
<212>  PRT
<213>  Mus sp.

<400>  6

Gln Gln Ile Asn Glu Asp Pro Phe Thr
1               5


<210>  7
<211>  5
<212>  PRT
<213>  Mus sp.

<400>  7

Asn Ser Gly Met Asn
1               5


<210>  8
<211>  17
<212>  PRT
<213>  Mus sp.

<400>  8

Trp Ile Asn Thr Tyr Thr Gly Glu Pro Thr Tyr Ala Asp Asp Phe Lys
1               5                   10                  15


Gly


<210>  9
<211>  5
<212>  PRT
<213>  Mus sp.

<400>  9

Arg Gly Phe Val Tyr
1               5


<210>  10
<211>  15
<212>  PRT
<213>  Mus sp.

<400>  10

Arg Ala Ser Glu Ser Val Ala Ile Tyr Gly Asn Ser Phe Leu Lys
1               5                   10                  15


<210>  11
<211>  7
<212>  PRT
<213>  Mus sp.

<400>  11

Arg Ala Ser Asn Leu Glu Ser
1               5


<210>  12
<211>  9
<212>  PRT
<213>  Mus sp.

<400>  12

Gln Gln Ile Asn Glu Asp Pro Tyr Thr
1               5


<210>  13
<211>  5
<212>  PRT
<213>  Mus sp.

<400>  13

Asp Tyr Trp Met Gln
1               5


<210>  14
<211>  17
<212>  PRT
<213>  Mus sp.

<400>  14

Thr Ile Tyr Pro Gly Asp Gly Asp Thr Gly Tyr Ala Gln Lys Phe Lys
1               5                   10                  15


Gly

<210>  15
<211>  11
<212>  PRT
<213>  Mus sp.

<400>  15

Gly Asp Tyr Tyr Gly Ser Asn Ser Leu Asp Tyr
1               5                   10


<210>  16
<211>  11
<212>  PRT
<213>  Mus sp.

<400>  16

Lys Ala Ser Gln Asp Val Ser Thr Val Val Ala
1               5                   10


<210>  17
<211>  7
<212>  PRT
<213>  Mus sp.

<400>  17

Ser Ala Ser Tyr Arg Tyr Ile
1               5


<210>  18
<211>  9
<212>  PRT
<213>  Mus sp.

<400>  18

Gln Gln His Tyr Ser Pro Pro Tyr Thr
1               5


<210>  19
<211>  5
<212>  PRT
<213>  Mus sp.

<400>  19

Gly Ser Trp Met Asn
1               5


<210>  20
<211>  17
<212>  PRT
<213>  Mus sp.

<400>  20

Arg Ile Tyr Pro Gly Asp Gly Asp Ile Ile Tyr Asn Gly Asn Phe Arg
1               5                   10                  15

Asp

<210>  21
<211>  10
<212>  PRT
<213>  Mus sp.

<400>  21

Trp Gly Thr Phe Thr Pro Ser Phe Asp Tyr
1               5                   10

<210>  22
<211>  11
<212>  PRT
<213>  Mus sp.

<400>  22

Lys Ala Ser Gln Asp Val Val Thr Ala Val Ala
1               5                   10

<210>  23
<211>  7
<212>  PRT
<213>  Mus sp.

<400>  23

Ser Ala Ser His Arg Tyr Thr
1               5

<210>  24
<211>  9
<212>  PRT
<213>  Mus sp.

<400>  24

Gln Gln His Tyr Thr Thr Pro Thr Thr
1               5

<210>  25
<211>  5
<212>  PRT
<213>  Mus sp.

<400>  25

Ser Tyr Thr Leu Ser
1               5

<210>  26
<211>  17
<212>  PRT
<213>  Mus sp.

<400>  26

Thr Ile Ser Ile Gly Gly Arg Tyr Thr Tyr Tyr Pro Asp Ser Val Glu
1               5                   10                  15

Gly

<210>  27
<211>  8
<212>  PRT
<213>  Mus sp.

<400>  27

Asp Phe Asn Gly Tyr Ser Asp Phe
1               5

<210>  28
<211>  11
<212>  PRT
<213>  Mus sp.

<400>  28

Lys Ala Ser Gln Val Val Gly Ser Ala Val Ala
1               5                   10

<210>  29
<211>  7
<212>  PRT
<213>  Mus sp.

<400>  29

Trp Ala Ser Thr Arg His Thr
1               5

<210>  30
<211>  9
<212>  PRT
<213>  Mus sp.

<400>  30

Gln Gln Tyr Asn Ser Tyr Pro Tyr Thr
1               5

<210>  31
<211>  5
<212>  PRT

<213>  Mus sp.

<400>  31

Asn Phe Gly Met His
1               5


<210>  32
<211>  17
<212>  PRT
<213>  Mus sp.

<400>  32

Tyr Ile Arg Ser Gly Ser Gly Thr Ile Tyr Tyr Ser Asp Thr Val Lys
1               5                   10                  15


Gly




<210>  33
<211>  11
<212>  PRT
<213>  Mus sp.

<400>  33

Ser Tyr Tyr Asp Phe Gly Ala Trp Phe Ala Tyr
1               5                   10


<210>  34
<211>  11
<212>  PRT
<213>  Mus sp.

<400>  34

Lys Ala Ser Gln Asn Val Gly Thr Asn Val Ala
1               5                   10


<210>  35
<211>  7
<212>  PRT
<213>  Mus sp.

<400>  35

Ser Ala Ser Ser Arg Tyr Ser
1               5


<210>  36
<211>  9
<212>  PRT
<213>  Mus sp.

<400>  36

Gln Gln Tyr Asn Ser Tyr Pro Leu Thr
1               5

<210>  37
<211>  336
<212>  DNA
<213>  Mus sp.

<220>
<221>  CDS
<222>  (1)..(336)

<400>  37
aac att gtg ctg acc caa tct cca gct tct ttg gct gtg tct ctt ggg          48
Asn Ile Val Leu Thr Gln Ser Pro Ala Ser Leu Ala Val Ser Leu Gly
1               5                   10                  15

cag agg gcc acc ata tcc tgc aga gcc agt gaa agt gtt gag att tat          96
Gln Arg Ala Thr Ile Ser Cys Arg Ala Ser Glu Ser Val Glu Ile Tyr
            20                  25                  30

ggc aat ggt ttt atg aac tgg ttc cag cag aaa cca gga cag cca ccc         144
Gly Asn Gly Phe Met Asn Trp Phe Gln Gln Lys Pro Gly Gln Pro Pro
        35                  40                  45

aaa ctc ctc atc tat cgt gca tcc aac cta gaa tct ggg atc cct gcc         192
Lys Leu Leu Ile Tyr Arg Ala Ser Asn Leu Glu Ser Gly Ile Pro Ala
    50                  55                  60

agg ttc agt ggc agt ggg tct agg aca gag ttc acc ctc acc att gat         240
Arg Phe Ser Gly Ser Gly Ser Arg Thr Glu Phe Thr Leu Thr Ile Asp
65                  70                  75                  80

cct gtg gag gct gat gat gtt gca acc tat tac tgt caa caa att aat         288
Pro Val Glu Ala Asp Asp Val Ala Thr Tyr Tyr Cys Gln Gln Ile Asn
                85                  90                  95

gag gat cca ttc acg ttc ggc tcg ggg aca aag ttg gaa ata aaa cgg         336
Glu Asp Pro Phe Thr Phe Gly Ser Gly Thr Lys Leu Glu Ile Lys Arg
            100                 105                 110

<210>  38
<211>  112
<212>  PRT
<213>  Mus sp.

<400>  38

Asn Ile Val Leu Thr Gln Ser Pro Ala Ser Leu Ala Val Ser Leu Gly
1               5                   10                  15

Gln Arg Ala Thr Ile Ser Cys Arg Ala Ser Glu Ser Val Glu Ile Tyr
            20                  25                  30

Gly Asn Gly Phe Met Asn Trp Phe Gln Gln Lys Pro Gly Gln Pro Pro
        35                  40                  45

Lys Leu Leu Ile Tyr Arg Ala Ser Asn Leu Glu Ser Gly Ile Pro Ala
    50                  55                  60

Arg Phe Ser Gly Ser Gly Ser Arg Thr Glu Phe Thr Leu Thr Ile Asp
65                  70                  75                  80

Pro Val Glu Ala Asp Asp Val Ala Thr Tyr Tyr Cys Gln Gln Ile Asn
                85                  90                  95

Glu Asp Pro Phe Thr Phe Gly Ser Gly Thr Lys Leu Glu Ile Lys Arg
            100                 105                 110

<210>  39
<211>  336
<212>  DNA
<213>  Mus sp.

<220>
<221>  CDS
<222>  (1)..(336)

<400>  39
gac att gta ctg acc caa tct cca gct tct ttg gct gtg tct cta ggg      48
Asp Ile Val Leu Thr Gln Ser Pro Ala Ser Leu Ala Val Ser Leu Gly
1               5                   10                  15

cag agg gcc acc ata tcc tgc aga gcc agt gag agt gtt gct att tat      96
Gln Arg Ala Thr Ile Ser Cys Arg Ala Ser Glu Ser Val Ala Ile Tyr
            20                  25                  30

ggc aat agt ttt ctg aaa tgg ttc cag cag aaa ccg gga cag cca ccc      144
Gly Asn Ser Phe Leu Lys Trp Phe Gln Gln Lys Pro Gly Gln Pro Pro
        35                  40                  45

aaa ctc ctc atc tat cgt gca tcc aac cta gaa tct ggg atc cct gcc      192
Lys Leu Leu Ile Tyr Arg Ala Ser Asn Leu Glu Ser Gly Ile Pro Ala
    50                  55                  60

agg ttc agt ggc agt ggg tct ggg aca gac ttc acc ctc acc att aat      240
Arg Phe Ser Gly Ser Gly Ser Gly Thr Asp Phe Thr Leu Thr Ile Asn
65                  70                  75                  80

cct gtg gag gct gat gat gtt gca acc tat tac tgt cag caa att aat      288
Pro Val Glu Ala Asp Asp Val Ala Thr Tyr Tyr Cys Gln Gln Ile Asn
                85                  90                  95

gag gat ccg tac acg ttc gga ggg ggg acc aag ctg gaa ata aaa cgg      336
Glu Asp Pro Tyr Thr Phe Gly Gly Gly Thr Lys Leu Glu Ile Lys Arg
            100                 105                 110

<210>  40
<211>  112
<212>  PRT
<213>  Mus sp.

<400>  40

Asp Ile Val Leu Thr Gln Ser Pro Ala Ser Leu Ala Val Ser Leu Gly
1               5                   10                  15

Gln Arg Ala Thr Ile Ser Cys Arg Ala Ser Glu Ser Val Ala Ile Tyr
            20                  25                  30

Gly Asn Ser Phe Leu Lys Trp Phe Gln Gln Lys Pro Gly Gln Pro Pro
        35                  40                  45

Lys Leu Leu Ile Tyr Arg Ala Ser Asn Leu Glu Ser Gly Ile Pro Ala
    50                  55                  60

Arg Phe Ser Gly Ser Gly Ser Gly Thr Asp Phe Thr Leu Thr Ile Asn
65                  70                  75                  80

Pro Val Glu Ala Asp Asp Val Ala Thr Tyr Tyr Cys Gln Gln Ile Asn
                85                  90                  95

Glu Asp Pro Tyr Thr Phe Gly Gly Gly Thr Lys Leu Glu Ile Lys Arg
            100                 105                 110

<210>  41
<211>  324
<212>  DNA
<213>  Mus sp.

<220>
<221>  CDS
<222>  (1)..(324)

<400>  41
gac att gtg atg gcc cag tct cac aaa ttc atg tcc aca tca gtt gga      48
Asp Ile Val Met Ala Gln Ser His Lys Phe Met Ser Thr Ser Val Gly
1               5                   10                  15

gac agg gtc agc atc acc tgc aag gcc agt cag gat gtg agt act gtt      96
Asp Arg Val Ser Ile Thr Cys Lys Ala Ser Gln Asp Val Ser Thr Val
            20                  25                  30

gtg gcc tgg tat caa cag aaa cca gga caa tct cct aaa cga ctg att      144
Val Ala Trp Tyr Gln Gln Lys Pro Gly Gln Ser Pro Lys Arg Leu Ile
        35                  40                  45

tac tcg gca tcc tat cgg tat att gga gtc cct gat cgc ttc act ggc      192
Tyr Ser Ala Ser Tyr Arg Tyr Ile Gly Val Pro Asp Arg Phe Thr Gly
    50                  55                  60

agt gga tct ggg acg gat ttc act ttc acc atc agc agt gtg cag gct      240
Ser Gly Ser Gly Thr Asp Phe Thr Phe Thr Ile Ser Ser Val Gln Ala
65                  70                  75                  80

gaa gac ctg gca gtt tat tac tgt cag caa cat tat agt cct ccg tac      288
Glu Asp Leu Ala Val Tyr Tyr Cys Gln Gln His Tyr Ser Pro Pro Tyr
                85                  90                  95

acg ttc gga ggg ggg acc aag ctg gaa ata aaa cgg                    324
Thr Phe Gly Gly Gly Thr Lys Leu Glu Ile Lys Arg
            100                 105

<210>  42
<211>  108
<212>  PRT
<213>  Mus sp.

<400>  42

Asp Ile Val Met Ala Gln Ser His Lys Phe Met Ser Thr Ser Val Gly
1               5                   10                  15

Asp Arg Val Ser Ile Thr Cys Lys Ala Ser Gln Asp Val Ser Thr Val
            20                  25                  30

Val Ala Trp Tyr Gln Gln Lys Pro Gly Gln Ser Pro Lys Arg Leu Ile
        35                  40                  45

Tyr Ser Ala Ser Tyr Arg Tyr Ile Gly Val Pro Asp Arg Phe Thr Gly
    50                  55                  60

Ser Gly Ser Gly Thr Asp Phe Thr Phe Thr Ile Ser Ser Val Gln Ala
65                  70                  75                  80

Glu Asp Leu Ala Val Tyr Tyr Cys Gln Gln His Tyr Ser Pro Pro Tyr
                85                  90                  95

Thr Phe Gly Gly Gly Thr Lys Leu Glu Ile Lys Arg
            100                 105

<210>  43
<211>  324
<212>  DNA
<213>  Mus sp.

<220>
<221>  CDS
<222>  (1)..(324)

<400>  43
gac att gtg atg acc cag tct cac aaa ttc ttg tcc aca tca gtt gga      48
Asp Ile Val Met Thr Gln Ser His Lys Phe Leu Ser Thr Ser Val Gly
1               5                   10                  15

gac agg gtc agt atc acc tgc aag gcc agt cag gat gtg gtt act gct      96
Asp Arg Val Ser Ile Thr Cys Lys Ala Ser Gln Asp Val Val Thr Ala
            20                  25                  30

gtt gcc tgg ttt caa cag aaa cca gga caa tct cca aaa cta ctg att      144
Val Ala Trp Phe Gln Gln Lys Pro Gly Gln Ser Pro Lys Leu Leu Ile
        35                  40                  45

tat tcg gca tcc cac cgg tac act gga gtc cct gat cgc ttc act ggc 192
Tyr Ser Ala Ser His Arg Tyr Thr Gly Val Pro Asp Arg Phe Thr Gly
    50                  55                  60

agt gga tct ggg aca gat ttc act ttc acc atc atc agt gtg cag gct 240
Ser Gly Ser Gly Thr Asp Phe Thr Phe Thr Ile Ile Ser Val Gln Ala
65                  70                  75                  80

gaa gac ctg gca gtt tat tac tgt caa caa cat tat act act ccc acg 288
Glu Asp Leu Ala Val Tyr Tyr Cys Gln Gln His Tyr Thr Thr Pro Thr
                85                  90                  95

acg ttc ggt gga ggc acc aag ctg gac ttc aga cgg 324
Thr Phe Gly Gly Gly Thr Lys Leu Asp Phe Arg Arg
            100                 105

<210> 44
<211> 108
<212> PRT
<213> Mus sp.

<400> 44

Asp Ile Val Met Thr Gln Ser His Lys Phe Leu Ser Thr Ser Val Gly
1               5                   10                  15

Asp Arg Val Ser Ile Thr Cys Lys Ala Ser Gln Asp Val Val Thr Ala
            20                  25                  30

Val Ala Trp Phe Gln Gln Lys Pro Gly Gln Ser Pro Lys Leu Leu Ile
        35                  40                  45

Tyr Ser Ala Ser His Arg Tyr Thr Gly Val Pro Asp Arg Phe Thr Gly
    50                  55                  60

Ser Gly Ser Gly Thr Asp Phe Thr Phe Thr Ile Ile Ser Val Gln Ala
65                  70                  75                  80

Glu Asp Leu Ala Val Tyr Tyr Cys Gln Gln His Tyr Thr Thr Pro Thr
                85                  90                  95

Thr Phe Gly Gly Gly Thr Lys Leu Asp Phe Arg Arg
            100                 105

<210> 45
<211> 324
<212> DNA
<213> Mus sp.

<220>
<221> CDS
<222> (1)..(324)

<400> 45

gac act gtg atg acc cag tct cac aaa ttc ata tcc aca tca gtt gga 48
Asp Thr Val Met Thr Gln Ser His Lys Phe Ile Ser Thr Ser Val Gly
1               5                   10                  15

gac agg gtc agc atc acc tgc aag gcc agt cag gtt gtg ggt agt gct 96
Asp Arg Val Ser Ile Thr Cys Lys Ala Ser Gln Val Val Gly Ser Ala
            20                  25                  30

gta gcc tgg tat caa cag aaa cca ggg caa tct cct aaa cta ctg att 144
Val Ala Trp Tyr Gln Gln Lys Pro Gly Gln Ser Pro Lys Leu Leu Ile
        35                  40                  45

tac tgg gca tcc acc cgg cac act gga gtc cct gat cgc ttc aca ggc 192
Tyr Trp Ala Ser Thr Arg His Thr Gly Val Pro Asp Arg Phe Thr Gly
    50                  55                  60

agt gga tct ggg aca gat ttc act ctc acc att agc aat gtg cag tct 240
Ser Gly Ser Gly Thr Asp Phe Thr Leu Thr Ile Ser Asn Val Gln Ser
65                  70                  75                  80

gaa gac ttg gca gat tat ttc tgt cag caa tat aac agc tat ccg tac 288
Glu Asp Leu Ala Asp Tyr Phe Cys Gln Gln Tyr Asn Ser Tyr Pro Tyr
                85                  90                  95

acg ttc gga ggg ggg acc aag ctg gaa ata aaa cgg 324
Thr Phe Gly Gly Gly Thr Lys Leu Glu Ile Lys Arg
            100                 105

<210> 46
<211> 108
<212> PRT
<213> Mus sp.

<400> 46

Asp Thr Val Met Thr Gln Ser His Lys Phe Ile Ser Thr Ser Val Gly
1               5                   10                  15

Asp Arg Val Ser Ile Thr Cys Lys Ala Ser Gln Val Val Gly Ser Ala
            20                  25                  30

Val Ala Trp Tyr Gln Gln Lys Pro Gly Gln Ser Pro Lys Leu Leu Ile
        35                  40                  45

Tyr Trp Ala Ser Thr Arg His Thr Gly Val Pro Asp Arg Phe Thr Gly
    50                  55                  60

Ser Gly Ser Gly Thr Asp Phe Thr Leu Thr Ile Ser Asn Val Gln Ser
65                  70                  75                  80

Glu Asp Leu Ala Asp Tyr Phe Cys Gln Gln Tyr Asn Ser Tyr Pro Tyr
                85                  90                  95

Thr Phe Gly Gly Gly Thr Lys Leu Glu Ile Lys Arg
            100                 105

<210>  47
<211>  324
<212>  DNA
<213>  Mus sp.

<220>
<221>  CDS
<222>  (1)..(324)

<400>  47
gac att gtg atg acc cag tct caa aaa ttc atg tcc aca tca gta gga      48
Asp Ile Val Met Thr Gln Ser Gln Lys Phe Met Ser Thr Ser Val Gly
1               5                   10                  15

gac agg gtc agc gtc acc tgc aag gcc agt cag aat gtg ggt act aat      96
Asp Arg Val Ser Val Thr Cys Lys Ala Ser Gln Asn Val Gly Thr Asn
            20                  25                  30

gtt gcc tgg tat caa cac aaa cca gga caa tcc cct aaa ata atg att      144
Val Ala Trp Tyr Gln His Lys Pro Gly Gln Ser Pro Lys Ile Met Ile
        35                  40                  45

tat tcg gcg tcc tcc cgg tac agt gga gtc cct gat cgc ttc aca ggc      192
Tyr Ser Ala Ser Ser Arg Tyr Ser Gly Val Pro Asp Arg Phe Thr Gly
    50                  55                  60

agt gga tct ggg aca ctt ttc act ctc acc atc aac aat gtg cag tct      240
Ser Gly Ser Gly Thr Leu Phe Thr Leu Thr Ile Asn Asn Val Gln Ser
65                  70                  75                  80

gaa gac ttg gca gag tat ttc tgt cag caa tat aac agc tat cct ctc      288
Glu Asp Leu Ala Glu Tyr Phe Cys Gln Gln Tyr Asn Ser Tyr Pro Leu
                85                  90                  95

acg ttc ggc tcg ggg aca aag ttg gaa ata aaa cgg                      324
Thr Phe Gly Ser Gly Thr Lys Leu Glu Ile Lys Arg
            100                 105

<210>  48
<211>  108
<212>  PRT
<213>  Mus sp.

<400>  48

Asp Ile Val Met Thr Gln Ser Gln Lys Phe Met Ser Thr Ser Val Gly
1               5                   10                  15

Asp Arg Val Ser Val Thr Cys Lys Ala Ser Gln Asn Val Gly Thr Asn
            20                  25                  30

Val Ala Trp Tyr Gln His Lys Pro Gly Gln Ser Pro Lys Ile Met Ile
        35                  40                  45

Tyr Ser Ala Ser Ser Arg Tyr Ser Gly Val Pro Asp Arg Phe Thr Gly
    50                  55                  60

Ser Gly Ser Gly Thr Leu Phe Thr Leu Thr Ile Asn Asn Val Gln Ser
65                  70                  75                  80

Glu Asp Leu Ala Glu Tyr Phe Cys Gln Gln Tyr Asn Ser Tyr Pro Leu
                85                  90                  95

Thr Phe Gly Ser Gly Thr Lys Leu Glu Ile Lys Arg
            100                 105

<210>  49
<211>  342
<212>  DNA
<213>  Mus sp.

<220>
<221>  CDS
<222>  (1)..(342)

<400>  49
cag atc cag ttg gtg cag tct gga cct gag ctg aag aag cct gga gag      48
Gln Ile Gln Leu Val Gln Ser Gly Pro Glu Leu Lys Lys Pro Gly Glu
1               5                   10                  15

aca gtc aag atc tcc tgc aag gct tct ggg tat acc ctc aca agc tac      96
Thr Val Lys Ile Ser Cys Lys Ala Ser Gly Tyr Thr Leu Thr Ser Tyr
            20                  25                  30

gga atg aac tgg gtg aag cag gct cca gga aag ggt tta aag tgg atg      144
Gly Met Asn Trp Val Lys Gln Ala Pro Gly Lys Gly Leu Lys Trp Met
        35                  40                  45

ggc tgg ata aac acc tac act gga gaa cca aca tat gct gat gac ttt      192
Gly Trp Ile Asn Thr Tyr Thr Gly Glu Pro Thr Tyr Ala Asp Asp Phe
    50                  55                  60

aag gga cgt ttt gcc ttc tct ttg gaa acc tct gcc agc act gcc ttt      240
Lys Gly Arg Phe Ala Phe Ser Leu Glu Thr Ser Ala Ser Thr Ala Phe
65                  70                  75                  80

ttg cag atc aac aac ctc aaa aat gag gac acg gct aca tat ttc tgt      288
Leu Gln Ile Asn Asn Leu Lys Asn Glu Asp Thr Ala Thr Tyr Phe Cys
                85                  90                  95

gta aga cgc ggg ttt gct tac tgg ggc caa ggg act ctg gtc act gtc      336
Val Arg Arg Gly Phe Ala Tyr Trp Gly Gln Gly Thr Leu Val Thr Val
            100                 105                 110

tct gca                                                              342
Ser Ala

<210>  50
<211>  114
<212>  PRT
<213>  Mus sp.

<400>  50

Gln Ile Gln Leu Val Gln Ser Gly Pro Glu Leu Lys Lys Pro Gly Glu
1               5                   10                  15

Thr Val Lys Ile Ser Cys Lys Ala Ser Gly Tyr Thr Leu Thr Ser Tyr
            20                  25                  30

Gly Met Asn Trp Val Lys Gln Ala Pro Gly Lys Gly Leu Lys Trp Met
        35                  40                  45

Gly Trp Ile Asn Thr Tyr Thr Gly Glu Pro Thr Tyr Ala Asp Asp Phe
    50                  55                  60

Lys Gly Arg Phe Ala Phe Ser Leu Glu Thr Ser Ala Ser Thr Ala Phe
65                  70                  75                  80

Leu Gln Ile Asn Asn Leu Lys Asn Glu Asp Thr Ala Thr Tyr Phe Cys
                85                  90                  95

Val Arg Arg Gly Phe Ala Tyr Trp Gly Gln Gly Thr Leu Val Thr Val
            100                 105                 110

Ser Ala

<210>  51
<211>  342
<212>  DNA
<213>  Mus sp.

<220>
<221>  CDS
<222>  (1)..(342)

<400>  51
cag atc cag ttg gtg cag tct gga cct gag ctg aag aag cct gga gag       48
Gln Ile Gln Leu Val Gln Ser Gly Pro Glu Leu Lys Lys Pro Gly Glu
1               5                   10                  15

aca gtc aag atc tcc tgc aag gct tct ggg tat acc ttc aca aac tct       96
Thr Val Lys Ile Ser Cys Lys Ala Ser Gly Tyr Thr Phe Thr Asn Ser
            20                  25                  30

gga atg aac tgg gtg aag cag gct cca gga aag ggt tta aag tgg atg      144
Gly Met Asn Trp Val Lys Gln Ala Pro Gly Lys Gly Leu Lys Trp Met
        35                  40                  45

ggc tgg ata aac acc tac act gga gag ccg aca tat gct gat gac ttc      192
Gly Trp Ile Asn Thr Tyr Thr Gly Glu Pro Thr Tyr Ala Asp Asp Phe
    50                  55                  60

aag gga cgg ttt gcc ttc tct ttg gaa acc tct gcc agc tct gcc tat      240
Lys Gly Arg Phe Ala Phe Ser Leu Glu Thr Ser Ala Ser Ser Ala Tyr
65                  70                  75                  80

ttg cag atc agt aac ctc aaa aat gag gac acg gct aca tat ttc tgt 288
Leu Gln Ile Ser Asn Leu Lys Asn Glu Asp Thr Ala Thr Tyr Phe Cys
                85                  90                  95

gca aga agg ggt ttt gtt tac tgg ggc caa ggg act ctg gta act gtc 336
Ala Arg Arg Gly Phe Val Tyr Trp Gly Gln Gly Thr Leu Val Thr Val
            100                 105                 110

tct gca 342
Ser Ala

<210> 52
<211> 114
<212> PRT
<213> Mus sp.

<400> 52

Gln Ile Gln Leu Val Gln Ser Gly Pro Glu Leu Lys Lys Pro Gly Glu
1               5                   10                  15

Thr Val Lys Ile Ser Cys Lys Ala Ser Gly Tyr Thr Phe Thr Asn Ser
            20                  25                  30

Gly Met Asn Trp Val Lys Gln Ala Pro Gly Lys Gly Leu Lys Trp Met
        35                  40                  45

Gly Trp Ile Asn Thr Tyr Thr Gly Glu Pro Thr Tyr Ala Asp Asp Phe
    50                  55                  60

Lys Gly Arg Phe Ala Phe Ser Leu Glu Thr Ser Ala Ser Ser Ala Tyr
65                  70                  75                  80

Leu Gln Ile Ser Asn Leu Lys Asn Glu Asp Thr Ala Thr Tyr Phe Cys
                85                  90                  95

Ala Arg Arg Gly Phe Val Tyr Trp Gly Gln Gly Thr Leu Val Thr Val
            100                 105                 110

Ser Ala

<210> 53
<211> 360
<212> DNA
<213> Mus sp.

<220>
<221> CDS
<222> (1)..(360)

<400>  53
cag gtt cag ctc cag cag tct ggg gct gag ctg gca aga cct ggg act      48
Gln Val Gln Leu Gln Gln Ser Gly Ala Glu Leu Ala Arg Pro Gly Thr
1               5                   10                  15

tca gtg aag ttg tcc tgt aag gct tct ggc tac acc ttt act gac tac      96
Ser Val Lys Leu Ser Cys Lys Ala Ser Gly Tyr Thr Phe Thr Asp Tyr
            20                  25                  30

tgg atg cag tgg gta aaa cag agg cct gga cag ggt ctg gag tgg att      144
Trp Met Gln Trp Val Lys Gln Arg Pro Gly Gln Gly Leu Glu Trp Ile
        35                  40                  45

ggg act att tat cct gga gat ggt gat act ggg tac gct cag aag ttc      192
Gly Thr Ile Tyr Pro Gly Asp Gly Asp Thr Gly Tyr Ala Gln Lys Phe
    50                  55                  60

aag ggc aag gcc aca ttg act gcg gat aaa tcc tcc aaa aca gtc tac      240
Lys Gly Lys Ala Thr Leu Thr Ala Asp Lys Ser Ser Lys Thr Val Tyr
65                  70                  75                  80

atg cac ctc agc agt ttg gct tct gag gac tct gcg gtc tat tac tgt      288
Met His Leu Ser Ser Leu Ala Ser Glu Asp Ser Ala Val Tyr Tyr Cys
                85                  90                  95

gca aga ggg gat tac tac ggt agt aat tct ttg gac tat tgg ggt caa      336
Ala Arg Gly Asp Tyr Tyr Gly Ser Asn Ser Leu Asp Tyr Trp Gly Gln
            100                 105                 110

gga acc tca gtc acc gtc tcc tca                                      360
Gly Thr Ser Val Thr Val Ser Ser
        115                 120


<210>  54
<211>  120
<212>  PRT
<213>  Mus sp.

<400>  54

Gln Val Gln Leu Gln Gln Ser Gly Ala Glu Leu Ala Arg Pro Gly Thr
1               5                   10                  15


Ser Val Lys Leu Ser Cys Lys Ala Ser Gly Tyr Thr Phe Thr Asp Tyr
            20                  25                  30


Trp Met Gln Trp Val Lys Gln Arg Pro Gly Gln Gly Leu Glu Trp Ile
        35                  40                  45


Gly Thr Ile Tyr Pro Gly Asp Gly Asp Thr Gly Tyr Ala Gln Lys Phe
    50                  55                  60


Lys Gly Lys Ala Thr Leu Thr Ala Asp Lys Ser Ser Lys Thr Val Tyr
65                  70                  75                  80


Met His Leu Ser Ser Leu Ala Ser Glu Asp Ser Ala Val Tyr Tyr Cys
                85                  90                  95

Ala Arg Gly Asp Tyr Tyr Gly Ser Asn Ser Leu Asp Tyr Trp Gly Gln
            100                 105                 110

Gly Thr Ser Val Thr Val Ser Ser
        115                 120

<210>  55
<211>  357
<212>  DNA
<213>  Mus sp.

<220>
<221>  CDS
<222>  (1)..(357)

<400>  55
cag gtc cag tta cag caa tct gga cct gaa ctg gtg agg cct ggg gcc      48
Gln Val Gln Leu Gln Gln Ser Gly Pro Glu Leu Val Arg Pro Gly Ala
1               5                   10                  15

tca gtg aag att tcc tgc aaa act tct ggc tac gca ttc agt ggc tcc      96
Ser Val Lys Ile Ser Cys Lys Thr Ser Gly Tyr Ala Phe Ser Gly Ser
            20                  25                  30

tgg atg aac tgg gtg aag cag agg cct gga cag ggt cta gag tgg att      144
Trp Met Asn Trp Val Lys Gln Arg Pro Gly Gln Gly Leu Glu Trp Ile
        35                  40                  45

gga cgg att tat ccg gga gat gga gat atc att tac aat ggg aat ttc      192
Gly Arg Ile Tyr Pro Gly Asp Gly Asp Ile Ile Tyr Asn Gly Asn Phe
    50                  55                  60

agg gac aag gtc aca ctg tct gca gac aaa tcc tcc aac aca gcc tac      240
Arg Asp Lys Val Thr Leu Ser Ala Asp Lys Ser Ser Asn Thr Ala Tyr
65                  70                  75                  80

atg cag ctc agc agc ctg acc tct gtg gac tct gcg gtc tat ttt tgt      288
Met Gln Leu Ser Ser Leu Thr Ser Val Asp Ser Ala Val Tyr Phe Cys
                85                  90                  95

tcg aga tgg ggg aca ttt acg ccg agt ttt gac tat tgg ggc caa ggc      336
Ser Arg Trp Gly Thr Phe Thr Pro Ser Phe Asp Tyr Trp Gly Gln Gly
            100                 105                 110

acc act ctc aca gtc tcc tca                                          357
Thr Thr Leu Thr Val Ser Ser
        115

<210>  56
<211>  119
<212>  PRT
<213>  Mus sp.

<400>  56

Gln Val Gln Leu Gln Gln Ser Gly Pro Glu Leu Val Arg Pro Gly Ala
1               5                   10                  15

Ser Val Lys Ile Ser Cys Lys Thr Ser Gly Tyr Ala Phe Ser Gly Ser
            20                  25                  30

Trp Met Asn Trp Val Lys Gln Arg Pro Gly Gln Gly Leu Glu Trp Ile
        35                  40                  45

Gly Arg Ile Tyr Pro Gly Asp Gly Asp Ile Ile Tyr Asn Gly Asn Phe
    50                  55                  60

Arg Asp Lys Val Thr Leu Ser Ala Asp Lys Ser Ser Asn Thr Ala Tyr
65                  70                  75                  80

Met Gln Leu Ser Ser Leu Thr Ser Val Asp Ser Ala Val Tyr Phe Cys
                85                  90                  95

Ser Arg Trp Gly Thr Phe Thr Pro Ser Phe Asp Tyr Trp Gly Gln Gly
            100                 105                 110

Thr Thr Leu Thr Val Ser Ser
        115

<210>  57
<211>  351
<212>  DNA
<213>  Mus sp.

<220>
<221>  CDS
<222>  (1)..(351)

<400>  57
gac gtg aag ctg gtg gag tct ggg gga ggc tta gtg aag cct gga ggg        48
Asp Val Lys Leu Val Glu Ser Gly Gly Gly Leu Val Lys Pro Gly Gly
1               5                   10                  15

tcc ctg aaa ctc tcc tgt gaa gcc tct gga ttc act ttc agt agc tat        96
Ser Leu Lys Leu Ser Cys Glu Ala Ser Gly Phe Thr Phe Ser Ser Tyr
            20                  25                  30

acc ctg tct tgg gtt cgc cag act ccg gag acg agg ctg gag tgg gtc       144
Thr Leu Ser Trp Val Arg Gln Thr Pro Glu Thr Arg Leu Glu Trp Val
        35                  40                  45

gca acc att agt att ggt ggt cgc tac acc tat tat cca gac agt gtg       192
Ala Thr Ile Ser Ile Gly Gly Arg Tyr Thr Tyr Tyr Pro Asp Ser Val
    50                  55                  60

gag ggc cga ttc acc atc tcc aga gac aat gcc aag aac acc ctg tac       240
Glu Gly Arg Phe Thr Ile Ser Arg Asp Asn Ala Lys Asn Thr Leu Tyr
65                  70                  75                  80

ctg caa atg aac agt ctg aag tct gag gac aca gcc atg tat tac tgt       288
Leu Gln Met Asn Ser Leu Lys Ser Glu Asp Thr Ala Met Tyr Tyr Cys

85                  90                  95

aca aga gat ttt aat ggt tac tct gac ttc tgg ggc caa ggc acc act          336
Thr Arg Asp Phe Asn Gly Tyr Ser Asp Phe Trp Gly Gln Gly Thr Thr
            100                 105                 110

ctc aca gtc tcc tca                                                      351
Leu Thr Val Ser Ser
        115

<210>  58
<211>  117
<212>  PRT
<213>  Mus sp.

<400>  58

Asp Val Lys Leu Val Glu Ser Gly Gly Gly Leu Val Lys Pro Gly Gly
1               5                   10                  15

Ser Leu Lys Leu Ser Cys Glu Ala Ser Gly Phe Thr Phe Ser Ser Tyr
            20                  25                  30

Thr Leu Ser Trp Val Arg Gln Thr Pro Glu Thr Arg Leu Glu Trp Val
        35                  40                  45

Ala Thr Ile Ser Ile Gly Gly Arg Tyr Thr Tyr Tyr Pro Asp Ser Val
    50                  55                  60

Glu Gly Arg Phe Thr Ile Ser Arg Asp Asn Ala Lys Asn Thr Leu Tyr
65                  70                  75                  80

Leu Gln Met Asn Ser Leu Lys Ser Glu Asp Thr Ala Met Tyr Tyr Cys
                85                  90                  95

Thr Arg Asp Phe Asn Gly Tyr Ser Asp Phe Trp Gly Gln Gly Thr Thr
            100                 105                 110

Leu Thr Val Ser Ser
        115

<210>  59
<211>  360
<212>  DNA
<213>  Mus sp.

<220>
<221>  CDS
<222>  (1)..(360)

<400>  59
aat gta cag ctg gta gag tct ggg gga ggc tta gtg cag cct gga ggg          48
Asn Val Gln Leu Val Glu Ser Gly Gly Gly Leu Val Gln Pro Gly Gly

1               5                   10                  15

tcc cgg aaa ctc tcc tgt gca gcc tct gga ttc act ttc agt aac ttt      96
Ser Arg Lys Leu Ser Cys Ala Ala Ser Gly Phe Thr Phe Ser Asn Phe
            20                  25                  30

gga atg cac tgg gtt cgt cag gct cca gag aag ggt ctg gag tgg gtc      144
Gly Met His Trp Val Arg Gln Ala Pro Glu Lys Gly Leu Glu Trp Val
        35                  40                  45

gca tac att cgt agt ggc agt ggt acc atc tac tat tca gac aca gtg      192
Ala Tyr Ile Arg Ser Gly Ser Gly Thr Ile Tyr Tyr Ser Asp Thr Val
    50                  55                  60

aag ggc cga ttc acc atc tcc aga gac aat ccc aag aac acc ctg ttc      240
Lys Gly Arg Phe Thr Ile Ser Arg Asp Asn Pro Lys Asn Thr Leu Phe
65                  70                  75                  80

ctg caa atg acc agt cta agg tct gag gac acg gcc atg tat tac tgt      288
Leu Gln Met Thr Ser Leu Arg Ser Glu Asp Thr Ala Met Tyr Tyr Cys
                85                  90                  95

gca aga tcc tac tat gat ttc ggg gcc tgg ttt gct tac tgg ggc caa      336
Ala Arg Ser Tyr Tyr Asp Phe Gly Ala Trp Phe Ala Tyr Trp Gly Gln
            100                 105                 110

ggg act ctg gtc act gtc tct gca                                      360
Gly Thr Leu Val Thr Val Ser Ala
        115                 120


<210>  60
<211>  120
<212>  PRT
<213>  Mus sp.

<400>  60

Asn Val Gln Leu Val Glu Ser Gly Gly Gly Leu Val Gln Pro Gly Gly
1               5                   10                  15


Ser Arg Lys Leu Ser Cys Ala Ala Ser Gly Phe Thr Phe Ser Asn Phe
            20                  25                  30


Gly Met His Trp Val Arg Gln Ala Pro Glu Lys Gly Leu Glu Trp Val
        35                  40                  45


Ala Tyr Ile Arg Ser Gly Ser Gly Thr Ile Tyr Tyr Ser Asp Thr Val
    50                  55                  60


Lys Gly Arg Phe Thr Ile Ser Arg Asp Asn Pro Lys Asn Thr Leu Phe
65                  70                  75                  80


Leu Gln Met Thr Ser Leu Arg Ser Glu Asp Thr Ala Met Tyr Tyr Cys
                85                  90                  95


Ala Arg Ser Tyr Tyr Asp Phe Gly Ala Trp Phe Ala Tyr Trp Gly Gln

100                 105                 110

Gly Thr Leu Val Thr Val Ser Ala
        115                 120

<210>  61
<211>  324
<212>  DNA
<213>  Homo sapiens

<220>
<221>  CDS
<222>  (1)..(324)

<400>  61
gat atc gta atg acc cag tcc cac ctg agt atg agt acc tcc ctg gga      48
Asp Ile Val Met Thr Gln Ser His Leu Ser Met Ser Thr Ser Leu Gly
1               5                   10                  15

gat cct gtg tca atc act tgc aag gcc tca cag gat gtg agc acc gtc      96
Asp Pro Val Ser Ile Thr Cys Lys Ala Ser Gln Asp Val Ser Thr Val
            20                  25                  30

gtt gct tgg tat cag cag aag ccc ggg caa tca ccc aga cgt ctc atc      144
Val Ala Trp Tyr Gln Gln Lys Pro Gly Gln Ser Pro Arg Arg Leu Ile
        35                  40                  45

tac tca gca tca tac cgt tac atc ggg gtg cct gac cga ttt act ggc      192
Tyr Ser Ala Ser Tyr Arg Tyr Ile Gly Val Pro Asp Arg Phe Thr Gly
    50                  55                  60

tct ggc gct ggc aca gat ttc acc ttt aca att agt tcc gtc cag gcc      240
Ser Gly Ala Gly Thr Asp Phe Thr Phe Thr Ile Ser Ser Val Gln Ala
65                  70                  75                  80

gaa gac ctg gcc gtg tac tac tgc cag cag cac tac agt ccc cca tac      288
Glu Asp Leu Ala Val Tyr Tyr Cys Gln Gln His Tyr Ser Pro Pro Tyr
                85                  90                  95

act ttc ggg gga ggg act aag ctc gaa atc aaa cgt                      324
Thr Phe Gly Gly Gly Thr Lys Leu Glu Ile Lys Arg
            100                 105

<210>  62
<211>  108
<212>  PRT
<213>  Homo sapiens

<400>  62

Asp Ile Val Met Thr Gln Ser His Leu Ser Met Ser Thr Ser Leu Gly
1               5                   10                  15

Asp Pro Val Ser Ile Thr Cys Lys Ala Ser Gln Asp Val Ser Thr Val
            20                  25                  30

Val Ala Trp Tyr Gln Gln Lys Pro Gly Gln Ser Pro Arg Arg Leu Ile

        35                  40                  45

Tyr Ser Ala Ser Tyr Arg Tyr Ile Gly Val Pro Asp Arg Phe Thr Gly
    50                  55                  60

Ser Gly Ala Gly Thr Asp Phe Thr Phe Thr Ile Ser Ser Val Gln Ala
65                  70                  75                  80

Glu Asp Leu Ala Val Tyr Tyr Cys Gln Gln His Tyr Ser Pro Pro Tyr
                85                  90                  95

Thr Phe Gly Gly Gly Thr Lys Leu Glu Ile Lys Arg
            100                 105

<210> 63
<211> 324
<212> DNA
<213> Homo sapiens

<220>
<221> CDS
<222> (1)..(324)

<400> 63
gac att gtt atg gct caa agc cat ctg tct atg agc aca tct ctg gga 48
Asp Ile Val Met Ala Gln Ser His Leu Ser Met Ser Thr Ser Leu Gly
1               5                   10                  15

gat cct gtg tcc atc act tgc aaa gcc agt caa gac gtg tct aca gtt 96
Asp Pro Val Ser Ile Thr Cys Lys Ala Ser Gln Asp Val Ser Thr Val
            20                  25                  30

gtt gca tgg tat caa cag aag cca ggc cag tca ccc aga cgg ctc att 144
Val Ala Trp Tyr Gln Gln Lys Pro Gly Gln Ser Pro Arg Arg Leu Ile
        35                  40                  45

tac tca gct tct tac cga tac atc ggg gtc cct gac aga ttt aca ggt 192
Tyr Ser Ala Ser Tyr Arg Tyr Ile Gly Val Pro Asp Arg Phe Thr Gly
    50                  55                  60

agt ggg gcc ggt act gac ttc act ttt act atc tca tcc gta caa gcc 240
Ser Gly Ala Gly Thr Asp Phe Thr Phe Thr Ile Ser Ser Val Gln Ala
65                  70                  75                  80

gaa gac ctg gca gta tat tac tgc cag caa cat tat tcc cca ccc tac 288
Glu Asp Leu Ala Val Tyr Tyr Cys Gln Gln His Tyr Ser Pro Pro Tyr
                85                  90                  95

aca ttc ggc ggg ggt act aag ctg gaa att aaa cgt 324
Thr Phe Gly Gly Gly Thr Lys Leu Glu Ile Lys Arg
            100                 105

<210> 64
<211> 108
<212> PRT
<213> Homo sapiens

<400>  64

Asp Ile Val Met Ala Gln Ser His Leu Ser Met Ser Thr Ser Leu Gly
1               5                   10                  15

Asp Pro Val Ser Ile Thr Cys Lys Ala Ser Gln Asp Val Ser Thr Val
            20                  25                  30

Val Ala Trp Tyr Gln Gln Lys Pro Gly Gln Ser Pro Arg Arg Leu Ile
        35                  40                  45

Tyr Ser Ala Ser Tyr Arg Tyr Ile Gly Val Pro Asp Arg Phe Thr Gly
    50                  55                  60

Ser Gly Ala Gly Thr Asp Phe Thr Phe Thr Ile Ser Ser Val Gln Ala
65                  70                  75                  80

Glu Asp Leu Ala Val Tyr Tyr Cys Gln Gln His Tyr Ser Pro Pro Tyr
                85                  90                  95

Thr Phe Gly Gly Gly Thr Lys Leu Glu Ile Lys Arg
            100                 105

<210>  65
<211>  360
<212>  DNA
<213>  Homo sapiens

<220>
<221>  CDS
<222>  (1)..(360)

<400>  65
cag gta cag ctc gtt cag tcc ggc gcc gag gta gct aag cct ggt act      48
tcc gta aaa ttg tcc tgt aag gct tcc ggg tac aca ttt aca gac tac      96
tgg atg cag tgg gta aaa cag cgg cca ggt cag ggc ctg gag tgg att      144
gga aca ata tat ccc ggc gac ggc gac aca ggc tat gcc cag aag ttt      192
caa ggc aag gca acc ctt act gct gat aaa tct tcc aag act gtc tac      240
atg cat ctg tct tcc ttg gca tct gag gat agc gct gtc tat tac tgt      288
gct agg ggg gac tac tat ggg tca aat tcc ctg gat tac tgg ggc cag      336
ggc acc agt gtc acc gtg agc agc                                      360

<210>  66
<211>  120
<212>  PRT
<213>  Homo sapiens

<400>  66

<210>  67
<211>  324
<212>  DNA
<213>  Homo sapiens

<220>
<221>  CDS
<222>  (1)..(324)

<400>  67
gac acc gtg atg acc cag tcc ccc tcc acc atc tcc acc tct gtg ggc      48

Asp Thr Val Met Thr Gln Ser Pro Ser Thr Ile Ser Thr Ser Val Gly
1               5                   10                  15

gac cgg gtg tcc atc acc tgt aag gcc tcc cag gtg gtg ggc tcc gcc      96
Asp Arg Val Ser Ile Thr Cys Lys Ala Ser Gln Val Val Gly Ser Ala
            20                  25                  30

gtg gcc tgg tat cag cag aag cct ggc cag tcc cct aag ctg ctg atc      144
Val Ala Trp Tyr Gln Gln Lys Pro Gly Gln Ser Pro Lys Leu Leu Ile
        35                  40                  45

tac tgg gcc tcc acc cgg cat acc ggc gtg cct gac cgg ttc acc ggc      192
Tyr Trp Ala Ser Thr Arg His Thr Gly Val Pro Asp Arg Phe Thr Gly
    50                  55                  60

tcc ggc agc ggc acc gac ttc acc ctg acc atc tcc aac gtg cag tcc      240
Ser Gly Ser Gly Thr Asp Phe Thr Leu Thr Ile Ser Asn Val Gln Ser
65                  70                  75                  80

gac gac ctg gcc gac tac ttc tgc cag cag tac aac tcc tac cct tac      288
Asp Asp Leu Ala Asp Tyr Phe Cys Gln Gln Tyr Asn Ser Tyr Pro Tyr
                85                  90                  95

acc ttt ggc ggc gga aca aag ctg gag atc aag cgt                      324
Thr Phe Gly Gly Gly Thr Lys Leu Glu Ile Lys Arg
            100                 105

<210>  68
<211>  108
<212>  PRT
<213>  Homo sapiens

<400>  68

Asp Thr Val Met Thr Gln Ser Pro Ser Thr Ile Ser Thr Ser Val Gly
1               5                   10                  15

Asp Arg Val Ser Ile Thr Cys Lys Ala Ser Gln Val Val Gly Ser Ala
            20                  25                  30

Val Ala Trp Tyr Gln Gln Lys Pro Gly Gln Ser Pro Lys Leu Leu Ile
        35                  40                  45

Tyr Trp Ala Ser Thr Arg His Thr Gly Val Pro Asp Arg Phe Thr Gly
    50                  55                  60

Ser Gly Ser Gly Thr Asp Phe Thr Leu Thr Ile Ser Asn Val Gln Ser
65                  70                  75                  80

Asp Asp Leu Ala Asp Tyr Phe Cys Gln Gln Tyr Asn Ser Tyr Pro Tyr
                85                  90                  95

Thr Phe Gly Gly Gly Thr Lys Leu Glu Ile Lys Arg
            100                 105

<210>  69
<211>  324
<212>  DNA
<213>  Homo sapiens

<220>
<221>  CDS
<222>  (1)..(324)

<400>  69
gac acc gtg atg acc cag tcc ccc tcc tcc atc tcc acc tcc atc ggc      48
Asp Thr Val Met Thr Gln Ser Pro Ser Ser Ile Ser Thr Ser Ile Gly
1               5                   10                  15

gac cgg gtg tcc atc acc tgt aag gcc tcc cag gtg gtg ggc tcc gcc      96
Asp Arg Val Ser Ile Thr Cys Lys Ala Ser Gln Val Val Gly Ser Ala
            20                  25                  30

gtg gcc tgg tat cag cag aag cct ggc cag tcc cct aag ctg ctg atc      144
Val Ala Trp Tyr Gln Gln Lys Pro Gly Gln Ser Pro Lys Leu Leu Ile
        35                  40                  45

tac tgg gcc tcc acc cgg cat acc ggc gtg cct gcc cgg ttc acc ggc      192
Tyr Trp Ala Ser Thr Arg His Thr Gly Val Pro Ala Arg Phe Thr Gly
    50                  55                  60

tcc ggc agc ggc acc gac ttc acc ctg acc atc tcc aac gtg cag tcc      240
Ser Gly Ser Gly Thr Asp Phe Thr Leu Thr Ile Ser Asn Val Gln Ser
65                  70                  75                  80

gag gac ctg gcc gac tac ttc tgc cag cag tac aac tcc tac cct tac      288
Glu Asp Leu Ala Asp Tyr Phe Cys Gln Gln Tyr Asn Ser Tyr Pro Tyr
                85                  90                  95

acc ttt ggc ggc gga aca aag ctg gag atc aag cgt                      324
Thr Phe Gly Gly Gly Thr Lys Leu Glu Ile Lys Arg
            100                 105

<210>  70
<211>  108
<212>  PRT
<213>  Homo sapiens

<400>  70

Asp Thr Val Met Thr Gln Ser Pro Ser Ser Ile Ser Thr Ser Ile Gly
1               5                   10                  15

Asp Arg Val Ser Ile Thr Cys Lys Ala Ser Gln Val Val Gly Ser Ala
            20                  25                  30

Val Ala Trp Tyr Gln Gln Lys Pro Gly Gln Ser Pro Lys Leu Leu Ile
        35                  40                  45

Tyr Trp Ala Ser Thr Arg His Thr Gly Val Pro Ala Arg Phe Thr Gly
    50                  55                  60

Ser Gly Ser Gly Thr Asp Phe Thr Leu Thr Ile Ser Asn Val Gln Ser
65                  70                  75                  80

Glu Asp Leu Ala Asp Tyr Phe Cys Gln Gln Tyr Asn Ser Tyr Pro Tyr
                85                  90                  95

Thr Phe Gly Gly Gly Thr Lys Leu Glu Ile Lys Arg
            100                 105

<210>  71
<211>  351
<212>  DNA
<213>  Homo sapiens

<220>
<221>  CDS
<222>  (1)..(351)

<400>  71
gag gtg cag ctg gtg gag tct ggc ggc gga ctg gtg aag cct ggc ggc      48
Glu Val Gln Leu Val Glu Ser Gly Gly Gly Leu Val Lys Pro Gly Gly
1               5                   10                  15

tcc ctg agg ctg tcc tgt gag gcc tcc ggc ttc acc ttc tcc tcc tac      96
Ser Leu Arg Leu Ser Cys Glu Ala Ser Gly Phe Thr Phe Ser Ser Tyr
            20                  25                  30

acc ctg tcc tgg gtg agg cag acc cct ggc aag ggc ctg gag tgg gtg      144
Thr Leu Ser Trp Val Arg Gln Thr Pro Gly Lys Gly Leu Glu Trp Val
        35                  40                  45

gcc acc atc tcc atc ggc ggc agg tac acc tac tac cct gac tcc gtg      192
Ala Thr Ile Ser Ile Gly Gly Arg Tyr Thr Tyr Tyr Pro Asp Ser Val
    50                  55                  60

aag ggc cgg ttc acc atc tcc cgg gac aac gcc aag aac acc ctg tac      240
Lys Gly Arg Phe Thr Ile Ser Arg Asp Asn Ala Lys Asn Thr Leu Tyr
65                  70                  75                  80

ctg cag atg aac tcc ctg aag tcc gag gac acc gcc atg tac tac tgt      288
Leu Gln Met Asn Ser Leu Lys Ser Glu Asp Thr Ala Met Tyr Tyr Cys
                85                  90                  95

acc cgg gac ttc aac ggc tac tcc gac ttc tgg ggc cag ggc acc aca      336
Thr Arg Asp Phe Asn Gly Tyr Ser Asp Phe Trp Gly Gln Gly Thr Thr
            100                 105                 110

ctg acc gtg tcc tcc                                                  351
Leu Thr Val Ser Ser
        115

<210>  72
<211>  117
<212>  PRT
<213>  Homo sapiens

<400>  72

Glu Val Gln Leu Val Glu Ser Gly Gly Gly Leu Val Lys Pro Gly Gly
1               5                   10                  15

Ser Leu Arg Leu Ser Cys Glu Ala Ser Gly Phe Thr Phe Ser Ser Tyr
            20                  25                  30

Thr Leu Ser Trp Val Arg Gln Thr Pro Gly Lys Gly Leu Glu Trp Val
        35                  40                  45

Ala Thr Ile Ser Ile Gly Gly Arg Tyr Thr Tyr Tyr Pro Asp Ser Val
    50                  55                  60

Lys Gly Arg Phe Thr Ile Ser Arg Asp Asn Ala Lys Asn Thr Leu Tyr
65                  70                  75                  80

Leu Gln Met Asn Ser Leu Lys Ser Glu Asp Thr Ala Met Tyr Tyr Cys
                85                  90                  95

Thr Arg Asp Phe Asn Gly Tyr Ser Asp Phe Trp Gly Gln Gly Thr Thr
            100                 105                 110

Leu Thr Val Ser Ser
        115

<210>  73
<211>  36
<212>  DNA
<213>  Mus sp.

<400>  73
ggaggatcca tagacagatg ggggtgtcgt tttggc                                36

<210>  74
<211>  32
<212>  DNA
<213>  Mus sp.

<400>  74
ggaggatccc ttgaccaggc atcctagagt ca                                    32

<210>  75
<211>  32
<212>  DNA
<213>  Mus sp.

<220>
<221>  misc_feature
<222>  (1)..(32)
<223>  mixed bases are defined as follows: H=A+T+C, S=G+C, Y=C+T,  K=
       G+T,  M=A+C,  R=A+G,  W=A+T,  V = A+C+G, N = A+C+G+T

<400>  75

cttccggaat tcsargtnma gctgsagsag tc                                    32

<210>  76
<211>  35
<212>  DNA
<213>  Mus sp.

<220>
<221>  misc_feature
<222>  (1)..(35)
<223>  mixed bases are defined as follows: H=A+T+C, S=G+C, Y=C+T,  K=
       G+T,  M=A+C,  R=A+G,  W=A+T,  V = A+C+G, N = A+C+G+T

<400>  76
cttccggaat tcsargtnma gctgsagsag tcwgg                                 35

<210>  77
<211>  31
<212>  DNA
<213>  Mus sp.

<220>
<221>  misc_feature
<222>  (1)..(31)
<223>  mixed bases are defined as follows: H=A+T+C, S=G+C, Y=C+T,  K=
       G+T,  M=A+C,  R=A+G,  W=A+T,  V = A+C+G, N = A+C+G+T

<400>  77
ggagctcgay attgtgmtsa cmcarwctmc a                                     31

<210>  78
<211>  46
<212>  DNA
<213>  Mus sp.

<400>  78
tatagagctc aagcttggat ggtgggaaga tggatacagt tggtgc                     46

<210>  79
<211>  21
<212>  DNA
<213>  Mus sp.

<400>  79
atggagtcac agattcaggt c                                                21

<210>  80
<211>  32
<212>  DNA
<213>  Mus sp.

<400>  80
ttttgaattc cagtaacttc aggtgtccac tc                                    32

<210>  81

<211>  17
<212>  PRT
<213>  Homo sapiens

<400>  81

Thr Ile Tyr Pro Gly Asp Gly Asp Thr Gly Tyr Ala Gln Lys Phe Gln
1               5                   10                  15

Gly